# EUROPÄISCHE PATENTSCHRIFT

(11) **EP 3 672 690 B1**
(45) Veröffentlichungstag und Bekanntmachung des Hinweises auf die Patenterteilung: **27.05.2026**
(21) Anmeldenummer: 18752493.9
(22) Anmeldetag: 14.08.2018
(51) Int. Cl.: A61Q 1/00, A61K 8/60, A61Q 17/00, A61Q 19/00, A61K 8/04

(54) **RHAMNOLIPIDDERIVATE ALS EMULGATOREN UND DISPERGIERHILFSMITTEL**
RHAMNOLIPID DERIVATIVES AS EMULSIFIERS AND A DISPERSANT
DÉRIVÉS DE RHAMNOLIPIDES COMME AGENTS ÉMULSIFIANTS ET AGENT DISPERSANT

(30) Priorität: 24.08.2017 EP 17187675
(43) Veröffentlichungstag der Anmeldung: 01.07.2020
(73) Patentinhaber: Evonik Operations GmbH, 45128 Essen (DE)
(72) Erfinder: LU, Xin, 45257 Essen (DE); NATTLAND, Sandra, 45359 Essen (DE); FRIEDRICH, Achim, 45529 Hattingen (DE); WENK, Hans Henning, 45470 Mülheim an der Ruhr (DE)
(74) Vertreter: Evonik Patent Association
(86) Internationale Anmeldenummer: PCT/EP2018/071974
(87) Internationale Veröffentlichungsnummer: WO 2019/038125

(56) Entgegenhaltungen:
- EP-A1- 3 023 431
- EP-A2- 1 889 623
- WO-A1-01/10447
- AHMAD MOHAMMAD ABDEL-MAWGOUD ET AL: "Rhamnolipids: diversity of structures, microbial origins and roles", APPLIED MICROBIOLOGY AND BIOTECHNOLOGY, SPRINGER, BERLIN, DE, vol. 86, no. 5, 25 March 2010 (2010-03-25), pages 1323 - 1336, XP019800002, ISSN: 1432-0614
- SHIDA MIAO ET AL: "Ethylation of Di-rhamnolipids: A Green Route to Produce Novel Sugar Fatty Acid Nonionic Surfactants", JOURNAL OF SURFACTANTS AND DETERGENTS, vol. 17, no. 6, 11 September 2014 (2014-09-11), DE, pages 1069 - 1080, XP055260723, ISSN: 1097-3958, DOI: 10.1007/s11743-014-1641-y

## Beschreibung

### Gebiet der Erfindung

Gegenstand der Erfindung ist eine Dispersion, insbesondere eine Emulsion, enthaltend mindestens ein Rhamnolipidderivat sowie die Verwendung der Rhamnolipidderivate als Emulgator oder Dispergierhilfsmittel.

### Stand der Technik

Rhamnolipide sind Tenside, die mittels Fermentation hergestellt werden können. Sie setzen sich aus ein bis zwei Rhamnose-Einheiten und ein bis drei, meist β-Hydroxy-Fettsäuren zusammen. Die Fettsäuren können gesättigt oder ungesättigt sein.

Die Variation in der Kettenlänge und Menge (Kongener) der Fettsäureanteile ist in einigen Veröffentlichungen beschrieben. (Howe et al., FEBS J. 2006; 273(22):5101-12; Abdel-Mawgoud et al., Appl Microbiol Biotechnol, 86, 2010; S. 1323-1336). Wenige kovalente Derivate der Fettsäureanteile von Rhamnolipiden sind bekannt.

Miao et al., Journal of Surfactants and Detergents, 17 (6), 2014; 1069-1080, beschreibt die Synthese von di-Rhamnolipidethylester durch die Veresterung mit Ethanol sowie die Eignung der Ester als nichtionisches Tensid.

WO2001010447 und EP1889623 offenbaren die pharmazeutischen und kosmetischen Anwendungen von Rhamnolipiden und kurzkettigen Rhamnolipidestern (C1-C6; Methyl- bis Hexylester, linear oder verzweigt), insbesondere bei der Wundheilung.

Aufgabe der Erfindung war es, hervorragende Emulgatoren oder Dispergierhilfsmittel bereitzustellen.

### Beschreibung der Erfindung

Überraschenderweise wurde gefunden, dass die im Folgenden beschriebenen Rhamnolipidderivate, welche durch eine relativ lange Kohlenstoffkette oder durch in Summe relativ viele Kohlenstoffatome aufweisende Substituenten derivatisiert sind, ausgezeichnete Emulgier- und Dispergierfähigkeiten aufweisen.

Gegenstand der vorliegenden Erfindung ist daher eine Dispersion enthaltend mindestens ein Rhamnolipidderivat wie in Anspruch 1 beschrieben.

### Gebiet der Erfindung

Gegenstand der Erfindung ist eine Dispersion, insbesondere eine Emulsion, enthaltend mindestens ein Rhamnolipidderivat sowie die Verwendung der Rhamnolipidderivate als Emulgator oder Dispergierhilfsmittel.

### Stand der Technik

Rhamnolipide sind Tenside, die mittels Fermentation hergestellt werden können. Sie setzen sich aus ein bis zwei Rhamnose-Einheiten und ein bis drei, meist β-Hydroxy-Fettsäuren zusammen. Die Fettsäuren können gesättigt oder ungesättigt sein.

Die Variation in der Kettenlänge und Menge (Kongener) der Fettsäureanteile ist in einigen Veröffentlichungen beschrieben. (Howe et al., FEBS J. 2006; 273(22):5101-12; Abdel-Mawgoud et al., Appl Microbiol Biotechnol, 86, 2010; S. 1323-1336). Wenige kovalente Derivate der Fettsäureanteile von Rhamnolipiden sind bekannt.

Miao et al., Journal of Surfactants and Detergents, 17 (6), 2014; 1069-1080, beschreibt die Synthese von di-Rhamnolipidethylester durch die Veresterung mit Ethanol sowie die Eignung der Ester als nichtionisches Tensid.

WO2001010447 und EP1889623 offenbaren die pharmazeutischen und kosmetischen Anwendungen von Rhamnolipiden und kurzkettigen Rhamnolipidestern (C1-C6; Methyl- bis Hexylester, linear oder verzweigt), insbesondere bei der Wundheilung.

Abdel-Mawgoud et al. offenbaren in Appl Microbiol Biotechnol. 2010 May, 86(5):1323-36 die Vielfältikgeit der Strukturen von Rhamnolipiden sowie deren mikrobiellen Ursprung und Rolle.

Miao et al. offenbaren in Journal of Surfactants and Detergents, 2010 November, 17(6):1069-1080 die Ethylierung von Di-Rhamnolipiden.

Aufgabe der Erfindung war es, hervorragende Emulgatoren oder Dispergierhilfsmittel bereitzustellen.

### Beschreibung der Erfindung

Überraschenderweise wurde gefunden, dass die im Folgenden beschriebenen Rhamnolipidderivate, welche durch eine relativ lange Kohlenstoffkette oder durch in Summe relativ viele Kohlenstoffatome aufweisende Substituenten derivatisiert sind, ausgezeichnete Emulgier- und Dispergierfähigkeiten aufweisen.

Gegenstand der vorliegenden Erfindung ist daher eine Dispersion enthaltend mindestens ein Rhamnolipidderivat wie in Anspruch 1 beschrieben.

Ein weiterer Gegenstand der Erfindung ist die Verwendung dieser Rhamnolipidderivate als Emulgator oder Dispergierhilfsmittel.

Ein Vorteil der in der vorliegenden Erfindung eingesetzten Rhamnolipidderivate liegt in ihrer guten Bioabbaubarkeit.

Noch ein Vorteil der vorliegenden Erfindung ist darin begründet, dass die Rhamnolipidderivate über die Wasserphase in die Emulsion eingearbeitet werden können.

Noch ein Vorteil der vorliegenden Erfindung liegt in der geringen hautreizenden Wirkung der eingesetzten Rhamnolipidderivate.

Noch ein Vorteil der vorliegenden Erfindung liegt in der geringen augenreizenden Wirkung der eingesetzten Rhamnolipidderivate.

Noch ein Vorteil der vorliegenden Erfindung liegt in der der guten Dispergierwirkung gegenüber dekorativen Pigmenten der eingesetzten Rhamnolipidderivate und der damit erzielbaren hohen Farbechtheit von bspw. Make-up-Produkten.

Noch ein Vorteil der vorliegenden Erfindung liegt in der guten Emulgierwirkung gegenüber organischen bzw. der guten Dispergierwirkung gegenüber anorganischen UV-Filtern.

Noch ein Vorteil der vorliegenden Erfindung ist, dass nach Anwendung der erfindungsgemäßen Emulsionen die Haut weniger stark entfettet wird.

Noch ein Vorteil der vorliegenden Erfindung ist, dass die erfindungsgemäßen Emulsionen unerwünschte Gerüche von Haut beseitigen können.

Ein weiterer Vorteil der vorliegenden Erfindung ist, dass die erfindungsgemäßen Emulsionen die Hautelastizität verbessern können.

Ein weiterer Vorteil der vorliegenden Erfindung ist, dass die erfindungsgemäßen Emulsionen schnell in die Haut einziehen und ein leichtes und angenehmes Hautgefühl hinterlassen.

Ein weiterer Vorteil der vorliegenden Erfindung ist, dass Emulsionen mit niedrigen Viskositäten realisiert werden können.

Ein weiterer Vorteil der vorliegenden Erfindung ist, dass die erfindungsgemäßen Emulsionen die Hautfeuchte verbessern.

Ein weiterer Vorteil der vorliegenden Erfindung ist, dass die erfindungsgemäßen Emulsionen im Zusammenspiel mit weiteren Tensiden oder nicht derivatisiertem mono- oder di-Rhamnolipid eine gute Schäumbarkeit aufweisen.

Ein weiterer Vorteil der vorliegenden Erfindung ist, dass die Rhamnolipidderivate auch als Coemulgatoren in Wasser-in-Öl-Emulsionen eingesetzt werden können.

Unter dem Begriff "Rhamnolipid" und "Rhamnolipidester" im Zusammenhang mit der vorliegenden Erfindung werden auch immer deren korrespondierenden Salze mitumfasst.

Unter dem Begriff "Rhamnolipid" und "Rhamnolipidamid" im Zusammenhang mit der vorliegenden Erfindung werden auch immer deren korrespondierenden Salze mitumfasst.

Unter dem Begriff "mono-Rhamnolipid" im Zusammenhang mit der vorliegenden Erfindung werden Verbindungen der unten gezeigten allgemeinen Formel (I) mit R³ = H oder deren Salze verstanden, bei denen n =0.

Distinkte Rhamnolipide werden gemäß folgender Nomenklatur abgekürzt:
Unter "diRL-CXCY" werden di-Rhamnolipide der allgemeinen Formel (I) mit R³ = H oder deren Salze verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Unter "monoRL-CXCY" werden mono-Rhamnolipide der allgemeinen Formel (I) mit R³ = H oder deren Salze verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Die verwendete Nomenklatur unterscheidet somit nicht zwischen "CXCY" und "CYCX".

Für Rhamnolipide mit m=0 wird entsprechend monoRL-CX bzw. diRL-CX verwendet.

Ist einer der oben genannten Indices X und/oder Y mit ":Z" versehen, so bedeutet dies, dass der jeweilige Rest R¹ und/oder R² = ein unverzweigter, unsubstituierter Kohlenwasserstoffrest mit X-3 bzw. Y-3 Kohlenstoffatomen aufweisend Z Doppelbindungen darstellt.

Analoge Nomenklatur wird für Rhamnolipidester in der Form di/monoRL-CXCY:Z-Ester eingesetzt.

Der "pH-Wert" im Zusammenhang mit der vorliegenden Erfindung ist definiert als der Wert, welcher für entsprechenden Stoff bei 25 °C nach fünf Minuten Rühren mit einer gemäß ISO 4319 (1977) kalibrierten pH-Elektrode gemessen wird.

Alle angegebenen Prozent (%) sind, wenn nicht anders angegeben, Massenprozent.

Gegenstand der vorliegenden Erfindung ist eine Dispersion enthaltend mindestens A) ein Rhamnolipidderivat ausgewählt aus der Gruppe bestehen aus Rhamnolipidester der allgemeinen Formel (I) und Rhamnolipidamide der allgemeinen Formel (II) 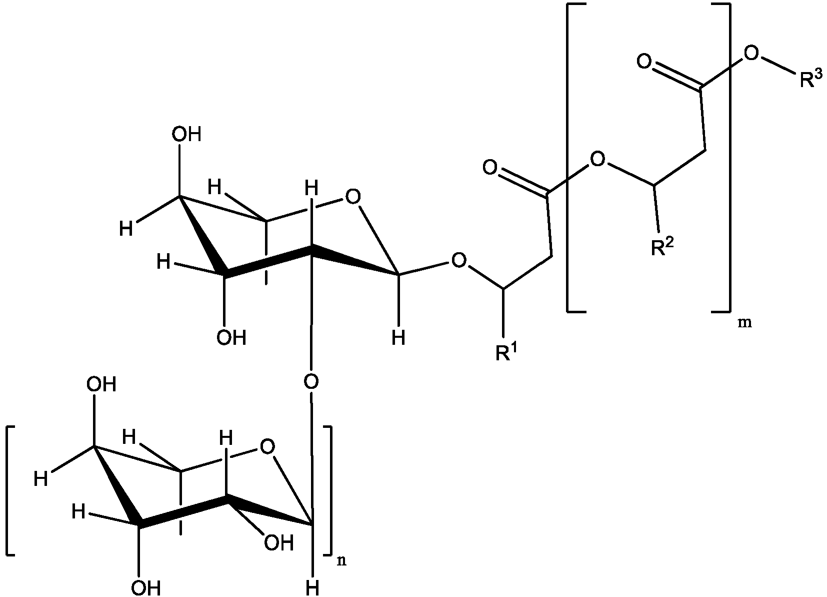 allgemeine Formel (I),
wobei
- m =: 2, 1 oder 0, insbesondere 1 oder 0,
- n =: 1 oder 0, insbesondere 1,
- R¹ =: organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
- R² =: unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12, und
- R³ =: aliphatischer Rest mit 7 bis 32 Kohlenstoffatomen, bevorzugt 8 bis 24 Kohlenstoffatomen, besonders bevorzugt 10 bis 22 Kohlenstoffatomen, welcher ausgewählt ist aus der Gruppe der Reste R³ direkt abgeleitet von R³OH = natürlicher Fettalkohol, 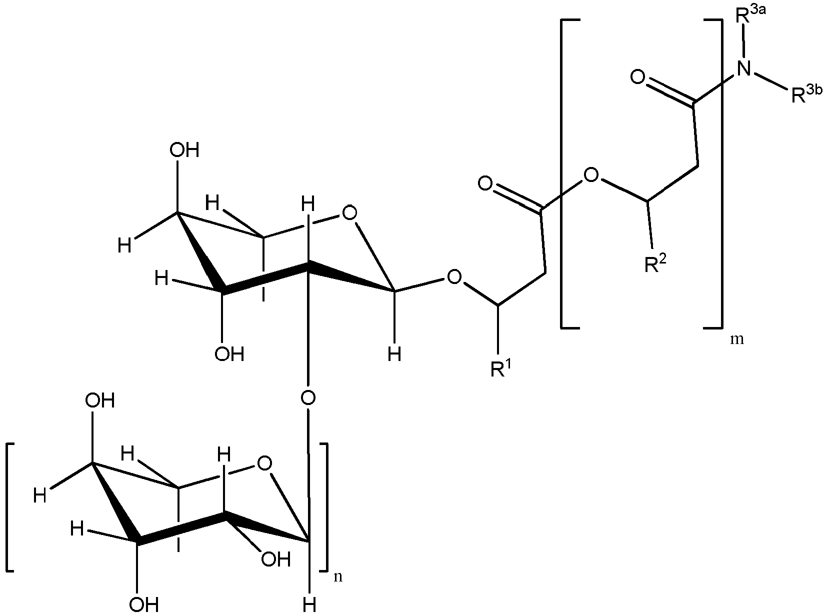
allgemeine Formel (II),
wobei
- m =: 2, 1 oder 0, insbesondere 1 oder 0,
- n =: 1 oder 0, insbesondere 1,
- R¹ =: organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
- R² =: unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
- R^{3a} =: organischer Rest mit 1 bis 32, bevorzugt 8 bis 24, besonders bevorzugt 10 bis 22, Kohlenstoffatomen,
- und R^{3b} =: organischer Rest mit 1 bis 32, bevorzugt 8 bis 24, besonders bevorzugt 10 bis 22, Kohlenstoffatomen oder H, bevorzugt H,
mit der Maßgabe, dass die Summe der in R^{3a} und R^{3b} enthaltenen Kohlenstoffatome 7 bis 44, bevorzugt 8 bis 24, besonders bevorzugt 10 bis 22, beträgt.

Das Rhamnolipidderivat ist erfindungsgemäß bevorzugt in der kontinuierlichen Phase der erfindungsgemäßen Dispersion enthalten.

Besonders bevorzugt erfindungsgemäß enthaltene Rhamnolipidester sind ausgewählt aus diRLC10C10-Ester, diRLC8C10-Ester, diRLC10C12-Ester, diRLC10C12:1-Ester sowie monoRLC10C10-Ester mit R³ = aliphatischer Rest mit 7 bis 32 Kohlenstoffatomen, bevorzugt 8 bis 24 Kohlenstoffatomen, besonders bevorzugt 10 bis 22 Kohlenstoffatomen.

enthaltene Rhamnolipidester sind dadurch gekennzeichnet, dass R³ ausgewählt ist aus der Gruppe der Reste R³ direkt abgeleitet von R³OH = natürlicher Fettalkohol.

In diesem Zusammenhang sind insbesondere Rhamnolipidester erfindungsgemäß bevorzugt ausgewählt aus diRLC10C10-Ester, diRLC8C10-Ester, diRLC10C12-Ester, diRLC10C12:1-Ester sowie monoRLC10C10-Ester.

Unter dem Begriff "natürlicher Fettalkohol" im Zusammenhang mit der vorliegenden Erfindung werden die durch Reduktion von natürlichen Triacylglycerole, Fettsäuren bzw. Fettsäuremethylester erhältlichen Alkohole verstanden; diese umfassen insbesondere lineare, gesättigte oder ungesättigte primäre Alkan-1-ole mit 8-32 Kohlenstoff-Atomen.

Ganz besonders bevorzugt erfindungsgemäß enthaltene Rhamnolipidester sind dadurch gekennzeichnet, dass R³ ausgewählt ist aus verzweigten oder linearen Alkylresten, bevorzugt mit 8 bis 24, insbesondere 10 bis 22, Kohlenstoffatomen. In diesem Zusammenhang sind insbesondere Rhamnolipidester bevorzugt ausgewählt aus diRLC10C10-Ester, diRLC8C10-Ester, diRLC10C12-Ester, diRLC10C12:1-Ester sowie monoRLC10C10-Ester.

Ganz besonders bevorzugt erfindungsgemäß enthaltene Rhamnolipidester sind dadurch gekennzeichnet, dass R³ ausgewählt ist aus der Gruppe umfassend, bevorzugt bestehend aus, Lauryl-, Myristyl-, Palmityl-, Stearyl-, Arachidyl- und Behenylresten. In diesem Zusammenhang sind insbesondere Rhamnolipidester erfindungsgemäß bevorzugt ausgewählt aus diRLC10C10-Ester, diRLC8C10-Ester, diRLC10C12-Ester, diRLC10C12:1-Ester sowie monoRLC10C10-Ester. Die erfindungsgemäß enthaltenen Rhamnolipidester sind bevorzugt Mischungszusammensetzungen von Rhamnolipidestern, die insbesondere dadurch gekennzeichnet sind, dass sie Mono- und Di-Rhamnolipidester enthalten.

Je nach Anwendung kann es bevorzugt sein, dass die erfindungsgemäß enthaltenen Mischungszusammensetzungen mehr Gewichtsprozente Mono-Rhamnolipidester als Di-Rhamnolipidester oder mehr Gewichtsprozente Di-Rhamnolipidester als Mono-Rhamnolipidester enthalten, wobei sich die Gewichtsprozente auf alle in der Mischungszusammensetzung enthaltenen Mono- und Di- Rhamnolipidester beziehen.

So können beispielsweise die erfindungsgemäß enthaltenen Mischungszusammensetzungen beispielsweise mehr als 60 Gew.-%, insbesondere mehr als 80 Gew.-%, oder gar mehr als 95 Gew.-%, Di-Rhamnolipidester enthalten, oder aber auch beispielsweise mehr als 60 Gew.-%, insbesondere mehr als 80 Gew.-%, oder gar mehr als 95 Gew.-%, Mono-Rhamnolipidester, wobei sich die Gewichtsprozente auf alle in der Mischungszusammensetzung enthaltenen Mono- und Di-Rhamnolipidester beziehen.

Die erfindungsgemäß enthaltenen Rhamnolipidester können durch ein Verfahren umfassend die Verfahrensschritte
A) Bereitstellen mindestens eines Rhamnolipides,
B) Umsetzen des Rhamnolipides mit mindestens einem Kopplungsreagens,
C) Umsetzen des durch Verfahrensschritt B) aktivierten Rhamnolipides mit einem Alkohol mit 1 bis 32, insbesondere 3 bis 32, Kohlenstoffatomen, und gegebenenfalls
D) Aufreinigen des Rhamnolipidesters,
hergestellt werden.

Verfahrensschritt A) wird nach den allgemein bekannten Verfahren des Standes der Technik durchgeführt, insbesondere unter Einsatz von gentechnisch veränderten Mikroorganismen, die bevorzugt Rhamnolipidsynthesegene überexpremieren, wobei diese Gene bevorzugt ausgewählt sind aus rhlA, rhlB und rhlC. Entsprechende Anleitung findet der Fachmann in z.B. US2014296168 und WO2012013554.

In Verfahrensschritt B) können als Kopplungsreagens beispielsweise Dicyclohexylcarbodiimid, Diisopropylcarbodiimid, 1-(3-Dimethylaminopropyl)-3-ethylcarbodiimidhydrochlorid, N-Cyclohexyl-N'-(2'-morpholinoethyl)carbodiimidmetho-p-toluolsulfonat, N-Benzyl-N'-3' dimethylaminopropylcarbodiimid-hydrochlorid, 1-Ethyl-3-(3-dimethylaminopropyl )carbodiimid N-Ethylcarbodiimid-hydrochlorid oder Carbonyldiimidazol eingesetzt werden.

Bevorzugt erfindungsgemäß enthaltene Rhamnolipidamide sind ausgewählt aus solchen Verbindungen der allgemeinen Formel (II), bei denen organischen Reste R^{3a} und R^{3b} ausgewählt sind aus gegebenenfalls ein- oder mehrfach ungesättigte Alkylreste, welche gegebenenfalls mindestens eine Amingruppe aufweisen.

Bevorzugt enthaltene Rhamnolipidamide sind ausgewählt aus Verbindungen der allgemeinen Formel (II), wobei
m = 2, 1 oder 0, insbesondere 1 oder 0,
n = 1 oder 0, insbesondere 1,
R¹ = gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, mit 2 bis 24, bevorzugt 5 bis 13 Kohlenstoffatomen, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
R² = gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest mit 2 bis 24, bevorzugt 5 bis 13 Kohlenstoffatomen, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12.

Bevorzugt enthaltene Rhamnolipidamide sind ausgewählt aus Verbindungen der allgemeinen Formel (II), wobei R^{3a} ausgewählt ist aus der Gruppe der Alkylreste, welche gegebenenfalls Amingruppe aufweisen, und R^{3b} = organischer Rest mit 1 bis 8 Kohlenstoffatomen, insbesondere Alkylrest, oder H, besonders bevorzugt H.

Besonders bevorzugt enthaltene Rhamnolipidamide sind ausgewählt aus diRLC10C10-Amiden, diC8C10-Amiden, diRLC10C12-Amiden, diRLC10C12:1-Amiden sowie monoRLC10C10-Amiden mit R^{3a} = organischer Rest mit 8 bis 24, bevorzugt 10 bis 22, Kohlenstoffatomen, insbesondere Alkylrest, und bevorzugt R^{3b} = H ist.

In einer alternative bevorzugten Ausführungsform sind die enthaltenen Rhamnolipidamide ausgewählt aus Verbindungen der allgemeinen Formel (II), wobei
R^{3a} = organischer Rest mit 10 bis 32 Kohlenstoffatomen, insbesondere Alkylrest,
und R^{3b} = organischer Rest mit 10 bis 32 Kohlenstoffatomen, insbesondere Alkylrest,
wobei es insbesondere bevorzugt ist, dass R^{3a} und R^{3b} unabhängig voneinander ausgewählt sind aus Alkylresten mit 10 bis 22 Kohlenstoffatomen.

In diesem Zusammenhang sind insbesondere Rhamnolipidamide bevorzugt ausgewählt aus diRLC10C10-Amiden, diC8C10-Amiden, diRLC10C12-Amiden, diRLC10C12:1-Amiden sowie monoRLC10C10-Amiden.

Ganz besonders bevorzugte Rhamnolipidamide sind dadurch gekennzeichnet, dass R^{3a} ausgewählt ist aus der Gruppe umfassend, bevorzugt bestehend aus, 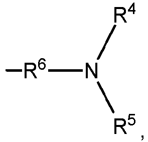 wobei
R⁴ und R⁵ = unabhängig voneinander gleicher oder verschiedener Alkylrest mit 1 bis 6, bevorzugt 1 bis 3, besonders bevorzugt 1, Kohlenstoffatomen,
R⁶ = eine Alkylengruppe mit 1 bis 6, bevorzugt, 2 bis 3 Kohlenstoffatomen,
   und 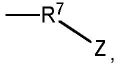 wobei
   R⁷ = eine Alkylengruppe mit 1 bis 22, bevorzugt, 2 bis 18, insbesondere 3 bis 8 Kohlenstoffatomen,
   Z = H, OH, OR⁸ mit
   R⁸ = Alkylrest mit 1 bis 6, bevorzugt 1 bis 3, besonders bevorzugt 1, Kohlenstoffatomen,
   und bevorzugt
   R^{3b} = H ist.

In diesem Zusammenhang sind insbesondere Rhamnolipidamide bevorzugt ausgewählt aus diRLC10C10-Amiden, diC8C10-Amiden, diRLC10C12-Amiden, diRLC10C12:1-Amiden sowie monoRLC10C10-Amiden.

In einer alternativen, bevorzugten Ausführungsform sind die erfindungsgemäß enthaltenen Rhamnolipidamide dadurch gekennzeichnet, dass sich der Rest -NR^{3a}R^{3b} ableitet von einem Amin NHR^{3a}R^{3b} ausgewählt aus Aminosäuren und Peptiden. In diesem Zusammenhang bevorzugte Aminosäuren sind ausgewählt aus den proteinogenen Aminosäuren. Weiterhin in diesem Zusammenhang bevorzugte Peptide sind ausgewählt aus Peptiden, welche aus proteinogenen Aminosäuren bestehen, insbesondere solche Peptiden umfassend 2 bis 20, insbesondere 4 bis 16, ganz besonders bevorzugt 4 bis 8 Aminosäuren.

Die erfindungsgemäß enthaltenen Rhamnolipidamide sind bevorzugt Mischungszusammensetzungen von Rhamnolipidamiden, die insbesondere dadurch gekennzeichnet sind, dass sie Mono- und Di- Rhamnolipidamide enthalten. Je nach Anwendung kann es bevorzugt sein, dass die erfindungsgemäß enthaltenen Mischungszusammensetzungen mehr Gewichtsprozente Mono-Rhamnolipidamide als Di-Rhamnolipidamide oder mehr Gewichtsprozente Di- Rhamnolipidamide als Mono-Rhamnolipidamide enthalten, wobei sich die Gewichtsprozente auf alle in der Mischungszusammensetzung enthaltenen Mono- und Di-Rhamnolipidamide beziehen. So können beispielsweise die erfindungsgemäß enthaltenen Mischungszusammensetzungen beispielsweise mehr als 60 Gew.-%, insbesondere mehr als 80 Gew.-%, oder gar mehr als 95 Gew.-%, Di-Rhamnolipidamide enthalten, oder aber auch beispielsweise mehr als 60 Gew.-%, insbesondere mehr als 80 Gew.-%, oder gar mehr als 95 Gew.-%, Mono-Rhamnolipidamide, wobei sich die Gewichtsprozente auf alle in der Mischungszusammensetzung enthaltenen Mono- und Di-Rhamnolipidamide beziehen.

Die erfindungsgemäß enthaltenen Rhamnolipidamide können durch ein Verfahren umfassend die Verfahrensschritte
A) Bereitstellen mindestens eines Rhamnolipides,
B) Umsetzen des Rhamnolipides mit mindestens einem Kopplungsreagens,
C) Umsetzen des durch Verfahrensschritt B) aktivierten Rhamnolipides mit einem Amin, und gegebenenfalls
D) Aufreinigen des Rhamnolipidamides
erhalten werden.

Verfahrensschritt A) wird nach den allgemein bekannten Verfahren des Standes der Technik durchgeführt, insbesondere unter Einsatz von gentechnisch veränderten Mikroorganismen, die bevorzugt Rhamnolipidsynthesegene überexpremieren, wobei diese Gene bevorzugt ausgewählt sind aus rhlA, rhlB und rhlC. Entsprechende Anleitung findet der Fachmann in z.B. US2014296168 und WO2012013554.

In Verfahrensschritt B) kann als Kopplungsreagens beispielsweise Dicyclohexylcarbodiimid, Diisopropylcarbodiimid, 1-(3-Dimethylaminopropyl)-3-ethylcarbodiimidhydrochlorid, N-Cyclohexyl-N'-(2'-morpholinoethyl)carbodiimidmetho-p-toluolsulfonat, N-Benzyl-N'-3' dimethylaminopropylcarbodiimid-hydrochlorid, 1-Ethyl-3-(3-dimethylaminopropyl)carbodiimid N-Ethylcarbodiimid-hydrochlorid und Carbonyldiimidazol eingesetzt werden. In Verfahrensschritt C) kann ein Katalysator wie beispielsweise N-Ethyldiisopropylamin, Trialkylamine, Pyridin, 4-Dimethylaminopyridin und Hydroxybenzotriazol, eingesetzt werden.

Erfindungsgemäß bevorzugte Dispersionen sind dadurch gekennzeichnet dass sie eine Emulsion, insbesondere eine Öl-in-Wasser Emulsion, darstellen.

Erfindungsgemäß bevorzugte Dispersionen sind dadurch gekennzeichnet dass sie
A) das Rhamnolipidderivat,
B) mindestens ein kosmetisches Öl und
C) Wasser
   enthalten.

### Komponente B) ist ein kosmetisches Öl.

Unter dem Begriff "kosmetisches Öl" im Zusammenhang mit der vorliegenden Erfindung sind mit Wasser nicht-mischbare Flüssigkeiten, welche zur Herstellung kosmetischer Formulierungen geeignet sind, zu verstehen. Mit Wasser nicht mischbar bedeutet im Zusammenhang mit der vorliegenden Erfindung, dass bei Raumtemperatur wässrige Mischungen von kosmetischen Ölen bei Ölkonzentrationen von 0,5 - 99,5 vol.-% bezogen auf die Gesamtmischung zu einer bereits mit dem menschlichen Auge wahrnehmbaren Trübung bzw. zur Ausbildung von zwei oder mehr Phasen führen. Des Weiteren sind im Zusammenhang mit der vorliegenden Erfindung kosmetische Öle bevorzugt dadurch gekennzeichnet, dass sie zu Wasser ein Grenzflächenspannung von > 5 mN/m aufweisen. Kosmetische Öle können zum Beispiel Oleochemie- oder Silikonchemie basiert sein.

Erfindungsgemäß bevorzugt sind in der erfindungsgemäßen Dispersion kosmetische Öle enthalten ausgewählt aus der Gruppe der Fettalkohole, Ester von linearen Fettsäuren mit linearen oder verzweigten Fettalkoholen, Ester von verzweigten Fettsäuren mit linearen oder verzweigten Fettalkoholen, Ester von linearen Fettsäuren mit unverzweigten oder verzweigten mehrwertigen Alkoholen, Ester von verzweigten Fettsäuren mit unverzweigten oder verzweigten mehrwertigen Alkoholen, Ester von linearen Fettsäuren mit unverzweigten oder verzweigten Alkoholen, Ester von verzweigten Fettsäuren mit unverzweigten oder verzweigten Alkoholen, Ester von Alkylhydroxycarbonsäuren mit linearen oder verzweigten Fettalkoholen. Des Weiteren Mono-, Di- oder Triglyceride in flüssiger oder fester Form. Des Weiteren Ester von Carbonsäuren, aromatischen Carbonsäuren oder Dicarbonsäuren mit linearen oder verzweigten Fettalkoholen, unverzweigten oder verzweigten mehrwertigen Alkoholen oder unverzweigten oder verzweigten Alkoholen. Des Weiteren lineare, cyclische oder verzweigte Kohlenwasserstoffe, mit oder ohne Substituenten, mit oder ohne Doppelbindungen. Des Weiteren pflanzliche Öle, Carbonate mit unverzweigten oder verzweigten Alkoholen, Carbonate mit unverzweigten oder verzweigten mehrwertigen Alkoholen, Carbonate mit linearen oder verzweigten Fettalkoholen. Des Weiteren Ether, mit oder ohne Alkoxygruppen, oder Silikonöle, mit oder ohne organische Modifizierung. Des Weiteren Mischungen dieser Öle in beliebigen Verhältnissen. Bevorzugt Ester von linearen Fettsäuren mit linearen oder verzweigten Fettalkoholen, Ester von verzweigten Fettsäuren mit linearen oder verzweigten Fettalkoholen, Ester von linearen Fettsäuren mit unverzweigten oder verzweigten mehrwertigen Alkoholen, Ester von verzweigten Fettsäuren mit unverzweigten oder verzweigten mehrwertigen Alkoholen, Ester von linearen Fettsäuren mit unverzweigten oder verzweigten Alkoholen, Ester von verzweigten Fettsäuren mit unverzweigten oder verzweigten Alkoholen. Des Weiteren Mono-, Di- oder Triglyceride in flüssiger oder fester Form. Des Weiteren Ester von Carbonsäuren, aromatischen Carbonsäuren oder Dicarbonsäuren mit linearen oder verzweigten Fettalkoholen, unverzweigten oder verzweigten mehrwertigen Alkoholen oder unverzweigten oder verzweigten Alkoholen. Des Weiteren lineare, cyclische oder verzweigte Kohlenwasserstoffe, mit oder ohne Substituenten, mit oder ohne Doppelbindungen. Des Weiteren pflanzliche Öle, Carbonate mit unverzweigten oder verzweigten Alkoholen, Carbonate mit unverzweigten oder verzweigten mehrwertigen Alkoholen, Carbonate mit linearen oder verzweigten Fettalkoholen, besonders bevorzugt lineare, cyclische oder verzweigte Kohlenwasserstoffe, mit oder ohne Substituenten, mit oder ohne Doppelbindungen. Des Weiteren Carbonate mit unverzweigten oder verzweigten Alkoholen, Carbonate mit unverzweigten oder verzweigten mehrwertigen Alkoholen, Carbonate mit linearen oder verzweigten Fettalkoholen. Ester von verzweigten Fettsäuren mit unverzweigten oder verzweigten mehrwertigen Alkoholen.

Erfindungsgemäß bevorzugte Emulsionen sind dadurch gekennzeichnet, dass
A) mind. ein Rhamnolipidderivat in einer Menge von 0,1 Gew.-% bis 10,0 Gew.-%, bevorzugt in einer Menge von 0,5 Gew.-% bis 7,0 Gew.-%, besonders bevorzugt in einer Menge von 1,0 Gew.-% bis 5,0 Gew.-%,
B) die Ölphase in einer Menge von 5,0 Gew.-% bis 79,9 Gew.-%, bevorzugt in einer Menge von 10,0 Gew.-% bis 50,0 Gew.-%, besonders bevorzugt in einer Menge von 12,0 Gew.-% bis 35,0 Gew.-%,
C) die Wasserphase in einer Menge von 20,0 Gew.-% bis 94,9 Gew.-%, bevorzugt in einer Menge von 50,0 Gew.-% bis 90,0 Gew.-%, besonders bevorzugt in einer Menge von 65,0 Gew.-% bis 88,0 Gew.-%,
enthalten ist, wobei sich die Gew.-% auf die Gesamtemulsion beziehen.

Ein weiterer Gegenstand der vorliegenden Erfindung ist die Verwendung der in den erfindungsgemäßen Dispersionen enthaltenen Rhamnolipidderivate als Emulgator oder Dispergierhilfsmittel. Die in den erfindungsgemäßen Dispersionen bevorzugt enthaltenen Rhamnolipidderivate werden bevorzugt im Rahmen der vorliegenden Erfindung als Emulgator oder Dispergierhilfsmittel verwendet.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1: Herstellung von di-Rhamnolipiden

Eine Fermentation mit einem rekombinanten Stamm *Pseudomonas putida* KT2440S *pBBR1MCS2-Plac-rhlABC-T-Ptac-rhlC-T* wurde durchgeführt. Die Konstruktion des Stamms wird beschrieben in der US2014296168. Die Vorkultur im Schüttelkolben wurde wie in WO2012013554 beschrieben durchgeführt. Für die Hauptkultur kam ebenfalls ein Mineralmedium (M9) zum Einsatz. Die Fermentation erfolgt in einem glukoselimitierten Fed-Batch - Verfahren in einem 2 Liter Fermenter. Die Glukose-Zufütterung wird anhand des Gelöstsauerstoffsignals reguliert. Der Sauerstoffpartialdruck der Fermentationsbrühe wurde bei 20 % Sättigung über die Rührerdrehzahl reguliert. Der pH-Wert wird über eine pH Elektrode und Zugabe von 2M Schwefelsäure bzw. einer 20 Gew.-% Ammoniaklösung auf 7 reguliert. Um das Überschäumen der Fermentationsbrühe zu verhindern, wurde der Entschäumer DOW Corning 1500 bei Bedarf zu dosiert. Die Fermentation wurde über 4 Tage bis zu einer Biotrockenmasse von 15 g/l geführt. Die Rhamnolipidkonzentration wurde über HPLC ermittelt und betrug 9,8 g/l. Nach Abtrennen der Zellen mittels Zentrifugation bei 10.000 g wurde die Fermentationsbrühe durch Zugabe konzentrierter H₂SO₄ auf einen pH-Wert von 3,1 eingestellt. Durch erneute Zentrifugation bei 10.000 g wurde ein pastöses Feststoff-Konzentrat mit einem RL-Anteil von 45 Gew.-% und mit einer Viskosität von > 10.000 mPas gewonnen. Unter ständigem Rühren wurde eine 50 gew.-%ige wässrige KOH Lösung zur pastösen Suspension des aufkonzentrierten Rhamnolipidpräzipitats gegeben und ein pH-Wert von 6 eingestellt. Hierbei kam es zur Verflüssigung der pastösen Masse, die mit einem starken Viskositätsabfall einherging. Aus der Suspension wurde eine klare Lösung. Durch Zugabe von Wasser wurde die Lösung auf einen Aktivgehalt von 35 Gew.-% eingestellt. Die Rhamnolipid-Reinheit betrug > 90 Gew.-% bezogen auf die Trockenmasse.

Mittels HPLC nachgewiesene Rhamnolipidspezies waren:

| | | |
|---|---|---|
| RL gesamt [%] (HPLC) | | **91** |
| | diRL-C8C10 | 13,9 |
| | monoRL-C8C10 | 0,51 |
| | diRL-C10C10 | 61,4 |
| | monoRL -C10C10 | 1,4 |
| | diRL-C10C12:1 | 5,9 |
| | diRL-C10C12 | 5,5 |
| | other RL | 2,2 |

### Beispiel 2: Herstellung von mono-Rhamnolipiden

Die wie oben beschrieben hergestellte, 35 Gew.-% Rhamnolipidlösung wurde durch Zugabe von Wasser auf 1 % verdünnt. Zwei Liter dieser Lösung wurden auf 50 °C erwärmt. Unter leichtem Rühren wurden 200 Units einer thermostabilen Rhamnosidase (ThermoActiveTM Rhamnosidase A, Prokazyme) zugegeben und die Reaktion über Nacht durchgeführt. Nach 20 h wurde eine Probe der Lösung mittels HPLC analysiert. Das di-Rhamnolipid war vollständig zu mono-Rhamnolipid und Rhamnose umgesetzt worden. Anschließend wurde das Enzym bei 80 °C eine Stunde inaktiviert. Dann wurde der gesamte Ansatz gefriergetrocknet. Das gefriergetrocknete Produkt wurde durch Wasserzugabe auf einen mono-Rhamnolipid Aktivgehalt von 35 Gew.-% eingestellt.

### Beispiel 3a: Synthese von di-Rhamnolipid-Behenylamid

Zur Aktivierung der Säurefunktion werden 25g di-Rhamnolipid (40 mmol) mit 6,25 ml Diisopropylcarbodiimid (40mmol) bei 55°C in THF gelöst. Ist eine Säurezahl von < 2 erreicht, werden 15,6 g Behenylamin (48mmol) zugegeben, sowie 1Gew% 4-Dimethylaminopyridin zur Katalyse. Das entstehende Reaktionswasser fördert die Bildung von N,N'-Diisopropylharnstoff als Nebenkomponente. Nach einer Reaktionzeit von 5 Stunden wird das Reaktionsgemisch am Rotationsversampfer getrocknet (45°C, < 300mbar), eine Aufreinigung erfolgt durch Ausschütteln mit Ethylacetat (1): Wasser (1) (2 x jeweils 20 ml) um den entstanden Harnstoff abzutrennen. Die Ethylacetatphase wird eingedampft (Rotationsverdampfer, 45°C, < 300mbar) und das Rhamnolipid-Behenylamid verbleibt als Feststoff.

Weitere Aufreinigung des Produkts kann mittels Säulenchromatographie erfolgen. Dazu dient Silica 60 gel (SIGMA Aldrich) als stationäre Phase und Ethylacetat (99) : Wasser (1) mit 1% Essigsäure als mobile Phase. Aus einer 5%igen Lösung des Rohprodukts werden Behenylamin-Reste, polare Neben- oder eventuelle Spaltprodukte entfernt. Zur sorgfältigen Auftrennung umfasst eine Fraktion 10ml bei einer Tropfgeschwindigkeit von 15 ml / min und einem Gesamtvolumen von 200 ml Ausgangslösung.

### Beispiel 3b : Synthese von mono-Rhamnolipiddioctylamid

Zur Aktivierung der Säurefunktion werden 20,2g mono-Rhamnolipid (40 mmol) mit 6,25 ml Diisopropylcarbodiimid bei 55°C in Toluol gelöst. Erreicht das Gemisch eine Säurezahl von < 2, werden 9,66 g Dioctylamin (40 mmol) zugefügt, ebenso 1-Gew% 4-Dimethylaminopyridin zur Katalyse. Eventuell nicht abreagiertes Kopplungsreagenz sollte zuvor durch Zugabe von 2 ml Wasser inaktiviert werden. Nach 10h Reaktionszeit erfolgt die Aufarbeitung. Das Reaktionsgemisch wird am Rotationsversampfer getrocknet (45°C, < 300mbar).

Zur Aufreinigung wird das Rohprodukt in Ethylacetat (2 Teile) gelöst und mit Wasser (1 Teil) bei pH 5,8 ausgeschüttelt. Der Rhamnolipidamide verbleibt in der Ethylacetatphase. (unlösliche Anteile abfiltrieren). Diese wird ebenfalls am Rotationsverdampfer getrocknet (45°C, <100mbar), um den Rhamnolipidamide zu erhalten.

### Beispiel 3c : Synthese von mono-Rhamnolipidstearylamid

Zur Aktivierung der Säurefunktion werden 20,2g mono-Rhamnolipid (40 mmol) mit 6,25 ml Diisopropylcarbodiimid bei 55°C in THF gelöst. Erreicht das Gemisch eine Säurezahl von < 2, werden 10,8 g Octadecylamin (40 mmol) zugefügt, ebenso 1-Gew% 4-Dimethylaminopyridin zur Katalyse. Eventuell nicht abreagiertes Kopplungsreagenz sollte zuvor durch Zugabe von 2 ml Wasser inaktiviert werden. Nach 10h Reaktionszeit erfolgt die Aufarbeitung. Das Reaktionsgemisch wird am Rotationsversampfer getrocknet (45°C, < 300mbar).

Zur Aufreinigung wird das Rohprodukt in Ethylacetat (2 Teile) gelöst und mit Wasser (1 Teil) bei pH 5,8 ausgeschüttelt. Der Rhamnolipidester verbleibt in der Ethylacetatphase. (unlösliche Anteile abfiltrieren). Diese wird ebenfalls am Rotationsverdampfer getrocknet (45°C, <100mbar), um den Rhamnolipidamide zu erhalten.

### Beispiel 4a: Synthese von di-Rhamnolipidstearylester

Zur Aktivierung der Säurefunktion werden 25g di-Rhamnolipid (40 mmol) aus Beispiel 1 mit 6,25 ml Diisopropylcarbodiimid bei 55°C in THF gelöst. Erreicht das Gemisch eine Säurezahl von < 2, werden 10,8g Stearylalkohol (40 mmol) zugefügt, ebenso 1-Gew% 4-Dimethylaminopyridin zur Katalyse. Eventuell nicht abreagiertes Kopplungsreagenz sollte zuvor durch Zugabe von 2 ml Wasser inaktiviert werden. Nach 10h Reaktionszeit erfolgt die Aufarbeitung. Das Reaktionsgemisch wird am Rotationsversampfer getrocknet (45°C, < 300mbar), eine Aufreinigung erfolgt durch Ausschütteln mit Ethylacetat (1): Wasser (1) in zwei Schritten mit je 250 ml. Der Rhamnolipidester verbleibt in der Ethylacetatphase. Diese wird ebenfalls am Rotationsverdampfer getrocknet (45°C, <100mbar), der zähflüssige Rhamnolipidester verbleibt.

Weitere Aufreinigung des Produkts kann mittels Säulenchromatographie erfolgen. Dazu dient Silica 60 Gel (SIGMA Aldrich) als stationäre Phase und Ethylacetat (99) : Wasser (1) mit 1% Essigsäure als mobile Phase. Aus einer 5%igen Lösung des Rhamnolipidester-Rohprodukts werden polare Neben- oder eventuelle Spaltprodukte entfernt. Zur sorgfältigen Auftrennung umfasst eine Fraktion 10ml bei einer Tropfgeschwindigkeit von 15 ml / min und einem Gesamtvolumen von 200 ml Ausgangslösung.

Analytische Bestimmung mittels HPLC erfolgte auf einer 50*3.0 mm column Poroshell 120 C18 (2.7µm) in 20mM NH₄ Formate in H₂O und MeCN bei 30°C für 35 min.

### Beispiel 4b : Synthese von mono-Rhamnolipidstearylester

Zur Aktivierung der Säurefunktion werden 20,2g mono-Rhamnolipid (40 mmol) mit 6,25 ml Diisopropylcarbodiimid bei 55°C in THF gelöst. Erreicht das Gemisch eine Säurezahl von < 2, werden 10,8g Stearylalkohol (40 mmol) zugefügt, ebenso 1-Gew% 4-Dimethylaminopyridin zur Katalyse. Eventuell nicht abreagiertes Kopplungsreagenz sollte zuvor durch Zugabe von 2 ml Wasser inaktiviert werden. Nach 10h Reaktionszeit erfolgt die Aufarbeitung. Das Reaktionsgemisch wird am Rotationsversampfer getrocknet (45°C, < 300mbar).

Zur Aufreinigung wird das Rohprodukt in Ethylacetat (2 Teile) gelöst und mit Wasser (1 Teil) bei pH 5,8 ausgeschüttelt. Der Rhamnolipidester verbleibt in der Ethylacetatphase. (unlösliche Anteile abfiltrieren). Diese wird ebenfalls am Rotationsverdampfer getrocknet (45°C, <100mbar), um den zähflüssige Rhamnolipidester zu erhalten.

### Beispiel 5a: nicht erfindungsgemäße Synthese von Di-Rhamnolipidethylestern

Die Synthese wurde nach der Vorschrift aus der Publikation Miao et al. European Journal of Lipid Science and Technology, 117, 2015; 156-1609, durchgeführt.

Hierfür wurde di-Rhamnolipid aus Beispiel 1 mit Ethanol und Schwefelsäure bei 0 °C wie in der Literatur beschreiben umgesetzt. Die nachgewiesene Ausbeute betrug weniger als 5%.

Dies ist unter anderem insbesondere dem Fehlen an Kopplungsreagenz zuzuschreiben.

### Beispiel 5b: nicht erfindungsgemäße Synthese von mono-Rhamnolipidethylestern

Die Synthese wurde nach der Vorschrift aus der Publikation Miao et al. European Journal of Lipid Science and Technology, 117, 2015; 156-1609, durchgeführt.

Hierzu wird mono-RL aus Beispiel 2 in Ethanol bei 0°C gerührt und die Veresterung mit Schwefelsäure katalysiert. Die nachgewiesene Ausbeute betrug auch hier weniger als 5%.

### Beispiel 6: Emulgierleistung

Alle Konzentrationen in den Anwendungsbeispielen sind in Gewichtsprozent angegeben. Zur Herstellung der Emulsionen wurden dem Fachmann bekannte übliche Homogenisierverfahren eingesetzt.

Die Herstellung der Emulsionen erfolgte daher typischerweise so, dass Öl- und Wasserphase auf 70 - 75°C erwärmt wurden. Anschließend wurde entweder die Ölphase in die Wassereingerührt oder Öl- und Wasserphase ohne Rühren zusammengegeben. Anschließend wurde mit einem geeigneten Homogenisator (z.B. Ultrathurrax) für ca. 1-2 Minuten homogenisiert.

Stabilisierende Polymere (z.B. Carbomere) werden bevorzugt als Öldispersion bei Temperaturen von 50 - 60°C in die Emulsion eingerührt. Anschließend wird kurz homogenisiert.

Zugabe weiterer Inhaltsstoffe (z.B. Konservierungsmittel, Wirkstoffe) erfolgte bevorzugt bei 40°C. Falls die Rezepturen mit organischen Säuren konserviert wurden, wurde der pH-Wert der Emulsionen auf ca. 5 eingestellt.

Diese Versuche sollen zeigen, dass die erfindungsgemäßen Rhamnolipidderivate Vorteile in Bezug auf Emulsionsstabilität im Vergleich zu unmodifizierten mono- oder di-Rhamnolipiden sowie zu kurzkettigen mono- oder di-Rhamnolipidestern aufweisen.

Zur Überprüfung der Lagerstabilität der Emulsionen wurden diese drei Monate bei Raumtemperatur, 40°C und 45°C gelagert. Zur Überprüfung der Kältestabilität wurde außerdem ein Monat lang bei -5°C gelagert und drei Gefrier-Tau-Zyklen von 25°C/-15°C/25°C durchgeführt. Deutlich Veränderungen im Erscheinungsbild oder der Konsistenz sowie insbesondere Öl- oder Wasserabscheidungen wurden als Kriterien für Instabilität gewichtet.

Vergleich der erfindungsgemäßen Rhamnolipidderivate als Emulgatoren 3a - c sowie 4a und b gegen die nicht derivatisierten Rhamnolipide nach Beispiel 1 und 2 sowie den nicht erfindungsgemäß derivatisierten Beispielen 5a und b in zwei Lotionen mit unterschiedlicher Zusammensetzung und Polarität der Ölphase.

| Rezeptur 1 | 1-1 | 1-2 | 1-3 | 1-4 | 1-5 |
|---|---|---|---|---|---|
| Erfindungsgemäßes Rhamnolipidderivat nach Bsp. 3a | 3,00% | - | - | - | - |
| Erfindungsgemäßes Rhamnolipidderivat nach Bsp. 3b | - | 3,00% | - | - | - |
| Erfindungsgemäßes Rhamnolipidderivat nach Bsp. 3c | - | - | 3,00% | - | - |
| Erfindungsgemäßes Rhamnolipidderivat nach Bsp. 4a | - | - | - | 3,00% | - |
| Erfindungsgemäßes Rhamnolipidderivat nach Bsp. 4b | - | - | - | - | 3,00% |
| Glyceryl Stearate | 0,50% | 0,50% | 0,50% | 0,50% | 0,50% |
| Stearic Acid | 0,50% | 0,50% | 0,50% | 0,50% | 0,50% |
| Caprylic/Capric Triglyceride | 5,50% | 5,50% | 5,50% | 5,50% | 5,50% |
| Ethylhexyl Palmitate | 6,30% | 6,30% | 6,30% | 6,30% | 6,30% |
| Wasser | ad 100% | ad 100% | ad 100% | ad 100% | ad 100% |
| Glycerin | 3,00% | 3,00% | 3,00% | 3,00% | 3,00% |
| Carbomer¹ | 0,20% | 0,20% | 0,20% | 0,20% | 0,20% |
| Sodium Hydroxide (10% ig in Wasser) | 0,60% | 0,60% | 0,60% | 0,60% | 0,60% |
| Phenoxyethanol; Ethylhexylglycerin² | 0,70% | 0,70% | 0,70% | 0,70% | 0,70% |
| | | | | | |
| Konsistenz nach Herstellung | dickflüssig | dünnflüssig | dickflüssig | dickflüssig | dickflüssig |
| Stabilität | Stabil | Stabil | Stabil | Stabil | Stabil |

| Rezeptur 1 | 1-6 | 1-7 | 1-8 | 1-9 | |
|---|---|---|---|---|---|
| Nicht erfindungsgemäßes, nichtderivatisiertes Mono-Rhamnolipid nach Bsp. 2 | 3,00% | - | - | - | |
| Nicht erfindungsgemäßes, nichtderivatisiertes Di-Rhamnolipid nach Bsp. 1 | - | 3,00% | - | - | |
| Nicht erfindungsgemäßes Mono-Rhamnolipidderivat nach Bsp. 5b | - | - | 3,00% | - | |

| | | | | | |
|---|---|---|---|---|---|
| ¹ TEGO^{®} Carbomer 141 (Evonik Nutrition & Care GmbH) ² Euxyl PE 9010 (Schülke & Mayr GmbH) | | | | | |

| | | | | |
|---|---|---|---|---|
| Nicht erfindungsgemäßes Di-Rhamnolipidderivat nach Bsp. 5a | - | - | - | 3,00% |
| Glyceryl Stearate | 0,50% | 0,50% | 0,50% | 0,50% |
| Stearic Acid | 0,50% | 0,50% | 0,50% | 0,50% |
| Caprylic/Capric Triglyceride | 5,50% | 5,50% | 5,50% | 5,50% |
| Ethylhexyl Palmitate | 6,30% | 6,30% | 6,30% | 6,30% |
| Wasser | ad 100% | ad 100% | ad 100% | ad 100% |
| Glycerin | 3,00% | 3,00% | 3,00% | 3,00% |
| Carbomer³ | 0,20% | 0,20% | 0,20% | 0,20% |
| Sodium Hydroxide (10% ig in Wasser) | 0,60% | 0,60% | 0,60% | 0,60% |
| Phenoxyethanol; Ethylhexylglycerin⁴ | 0,70% | 0,70% | 0,70% | 0,70% |
| | | | | |
| Konsistenz nach Herstellung | pastös | pastös | pastös | pastös |
| Stabilität | Phasens eparation nach 1 Tag bei RT | Phasens eparation nach 1 Tag bei RT | Phasens eparation nach 3 Tagen bei RT | Phasens eparation nach 3 Tagen bei RT |

| | | | | |
|---|---|---|---|---|
| ³ TEGO^{®} Carbomer 141 (Evonik Nutrition & Care GmbH) ⁴ Euxyl PE 9010 (Schülke & Mayr GmbH) | | | | |

| Rezeptur 2 | 2-1 | 2-2 | 2-3 | 2-4 | 2-5 |
|---|---|---|---|---|---|
| Erfindungsgemäßes Rhamnolipidderivat nach Bsp. 3a | 3,00% | - | - | - | - |
| Erfindungsgemäßes Rhamnolipidderivat nach Bsp. 3b | - | 3,00% | - | - | - |
| Erfindungsgemäßes Rhamnolipidderivat nach Bsp. 3c | - | - | 3,00% | - | - |
| Erfindungsgemäßes Rhamnolipidderivat nach Bsp. 4a | - | - | - | 3,00% | - |
| Erfindungsgemäßes Rhamnolipidderivat nach Bsp. 4b | - | - | - | - | 3,00% |
| Glyceryl Stearate | 0,50% | 0,50% | 0,50% | 0,50% | 0,50% |
| Stearic Acid | 0,50% | 0,50% | 0,50% | 0,50% | 0,50% |
| Ethylhexyl Palmitate | 6,30% | 6,30% | 6,30% | 6,30% | 6,30% |
| Paraffinum Perliquidum | 5,50% | 5,50% | 5,50% | 5,50% | 5,50% |
| Wasser | ad 100% | ad 100% | ad 100% | ad 100% | ad 100% |
| Glycerin | 3,00% | 3,00% | 3,00% | 3,00% | 3,00% |
| Carbomer¹ | 0,20% | 0,20% | 0,20% | 0,20% | 0,20% |
| Sodium Hydroxide (10% ig in Wasser) | 0,60% | 0,60% | 0,60% | 0,60% | 0,60% |
| Phenoxyethanol; Ethylhexylglycerin² | 0,70% | 0,70% | 0,70% | 0,70% | 0,70% |
| | | | | | |
| Konsistenz nach Herstellung | dickflüssig | dünnflüssig | dickflüssig | dickflüssig | dickflüssig |
| Stabilität | Stabil | Stabil | Stabil | Stabil | Stabil |

| Rezeptur 2 | 2-6 | 2-7 | 2-8 | 2-9 |
|---|---|---|---|---|
| Nicht erfindungsgemäßes, nichtderivatisiertes Mono-Rhamnolipid nach Bsp. 2 | 3,00% | - | - | - |
| Nicht erfindungsgemäßes, nichtderivatisiertes Di-Rhamnolipid nach Bsp. 1 | - | 3,00% | - | - |
| Nicht erfindungsgemäßes Mono-Rhamnolipidderivat nach Bsp. 5b | - | - | 3,00% | - |
| Nicht erfindungsgemäßes Di-Rhamnolipidderivat nach Bsp. 5a | - | - | - | 3,00% |
| Glyceryl Stearate | 0,50% | 0,50% | 0,50% | 0,50% |
| Stearic Acid | 0,50% | 0,50% | 0,50% | 0,50% |
| Ethylhexyl Palmitate | 6,30% | 6,30% | 6,30% | 6,30% |
| Paraffinum Perliquidum | 5,50% | 5,50% | 5,50% | 5,50% |
| Wasser | ad 100% | ad 100% | ad 100% | ad 100% |
| Glycerin | 3,00% | 3,00% | 3,00% | 3,00% |
| Carbomer¹ | 0,20% | 0,20% | 0,20% | 0,20% |
| Sodium Hydroxide (10% ig in Wasser) | 0,60% | 0,60% | 0,60% | 0,60% |
| Phenoxyethanol; Ethylhexylglycerin² | 0,70% | 0,70% | 0,70% | 0,70% |
| | | | | |
| Konsistenz nach Herstellung | pastös | pastös | pastös | pastös |
| Stabilität | Phasense paration nach 1 Tag bei RT | Phasense paration nach 1 Tag bei RT | Phasens eparation nach 5 Tagen bei RT | Phasens eparation nach 5 Tagen bei RT |

Während die Rezepturen mit den erfindungsgemäßen Emulgatoren 3a - *c sowie* 4a *und b* jeweils zu einer lagerstabilen Lotion führen, zeigen diejenigen mit den nicht erfindungsgemäßen Vergleichsemulgatoren aus den Beispielen 1, 2, 5a und 5b eine initial zu hohe Viskosität und starke Schwächen in der Lagerstabilität der Emulsion.

### Formulierungsbeispiele

Diese Beispiele sollen zeigen, dass die erfindungsgemäßen Rhamnolipidderivate in einer Vielzahl kosmetischer Formulierungen als Emulgatoren eingesetzt werden können.

Es ist darüber hinaus mit Hilfe der erfindungsgemäßen Rhamnolipidderivate möglich, Pigmente oder Festkörper stabil in Emulsionspräparate einzuarbeiten.

Weiterhin zeigen die Beispiele die gute Kompatibilität mit typischen Coemulgatoren, Ölen, Verdickern und Stabilisatoren sowie die gute Verträglichkeit mit emulsionsbelastenden Inhaltsstoffen wie UV-Filtern, antimikrobiellen Wirkstoffen oder kosmetischen Wirkstoffen.

### Lotionen mit niedrigem Emulgatorgehalt

| Rezeptur | 3-1 | 3-2 | 4-1 | 4-2 |
|---|---|---|---|---|
| Erfindungsgemäßes Rhamnolipidderivat nach Bsp. 4a | 1,00% | 0,50% | 1,00% | 0,50% |
| Cetearyl Glucoside⁵ | - | 0,50% | - | - |
| Polyglyceryl-6 Stearate;Polyglyceryl-6 Behenate⁶ | - | - | - | 1,00% |
| Glyceryl Stearate | 0,50% | 0,50% | 0,50% | 0,50% |
| Stearic Acid | 0,50% | 0,50% | 0,50% | 0,50% |
| Caprylic/Capric Triglyceride | 6,50% | 6,50% | | |
| Ethylhexyl Palmitate | 8,10% | 8,10% | 8,10% | 8,10% |
| Paraffinum Perliquidum | | | 6,50% | 6,50% |
| Wasser | ad 100% | ad 100% | ad 100% | ad 100% |
| Glycerin | 3,00% | 3,00% | 3,00% | 3,00% |
| Carbomer¹ | 0,20% | 0,20% | 0,20% | 0,20% |
| Sodium Hydroxide (10% ig in Wasser) | 0,60% | 0,60% | 0,60% | 0,60% |
| Phenoxyethanol; Ethylhexylglycerin² | 0,70% | 0,70% | 0,70% | 0,70% |

### Serum

| Rezeptur | 5-1 | 5-2 | 5-3 | 5-4 |
|---|---|---|---|---|
| Erfindungsgemäßes Rhamnolipidderivat nach Bsp. 3a | 2,00% | 1,00% | - | - |
| Erfindungsgemäßes Rhamnolipidderivat nach Bsp. 3c | - | - | 2,00% | 1,00% |
| Sorbitan Laurate; Polyglyceryl-4 Laurate; Dilauryl Citrate⁷ | - | 1,00% | - | 1,00% |
| Ethylhexyl Stearate | 7,00% | 7,00% | 7,00% | 7,00% |
| Octyldodecanol | 4,00% | 4,00% | 4,00% | 4,00% |
| Caprylic/Capric Triglyceride | 2,00% | 2,00% | 2,00% | 2,00% |
| Carbomer⁸ | 0,15% | 0,15% | 0,15% | 0,15% |
| Carbomer¹ | 0,15% | 0,15% | 0,15% | 0,15% |
| Xanthan Gum⁹ | 0,10% | 0,10% | 0,10% | 0,10% |
| Hydrolyzed Hyaluronic Acid¹⁰ | 0,10% | 0,10% | 0,10% | 0,10% |

| | | | | |
|---|---|---|---|---|
| ⁵ TEGO^{®} Care CG 90 (Evonik Nutrition & Care GmbH) ⁶ TEGO^{®} Care PBS 6 (Evonik Nutrition & Care GmbH) ⁷ TEGO^{®} Care LTP (Evonik Nutrition & Care GmbH) ⁸ TEGO^{®} Carbomer 140 (Evonik Nutrition & Care GmbH) ⁹ Keltrol CG-SFT (CP Kelco) ¹⁰ HyaCare^{®} 50 (Evonik Nutrition & Care GmbH) | | | | |

| | | | | |
|---|---|---|---|---|
| Ceteareth-25; Glycerin; Cetyl Alcohol; Behenic Acid; Cholesterol; Ceramide EOS; Ceramide NP; Ceramide NS; Ceramide AP; Caprooyl Phytosphingosine; Caprooyl Sphingosine)¹¹ | 1,00% | 1,00% | 1,00% | 1,00% |
| Glycerin | 3,00% | 3,00% | 3,00% | 3,00% |
| Wasser | ad 100% | ad 100% | ad 100% | ad 100% |
| Sodium Hydroxide (10% ig in Wasser) | 0,90% | 0,90% | 0,90% | 0,90% |
| Caprooyl Phytosphingosine¹² | 0,10% | 0,10% | 0,10% | 0,10% |
| Pentylene Glycol | 1,40% | 1,40% | 1,40% | 1,40% |
| Phenoxyethanol; Caprylyl Glycol¹³ | 1,00% | 1,00% | 1,00% | 1,00% |

### O/W Sonnenschutzlotion SPF 30

| Rezeptur | 6-1 | 6-2 | 6-3 | 6-4 |
|---|---|---|---|---|
| Erfindungsgemäßes Rhamnolipidderivat nach Bsp. 4a | 3,00% | 1,00% | - | - |
| Erfindungsgemäßes Rhamnolipidderivat nach Bsp. 4b | - | - | 3,00% | 1,00% |
| Cetearyl Glucoside³ | - | 1,00% | - | 1,00% |
| Phenoxyethyl Caprylate¹⁴ | 6,10% | 6,10% | 6,10% | 6,10% |
| Cetearyl Alcohol | 1,00% | 1,00% | 1,00% | 1,00% |
| Octocrylene | 4,80% | 4,80% | 4,80% | 4,80% |
| Ethylhexyl Triazone | 4,50% | 4,50% | 4,50% | 4,50% |
| Butyl Methoxydibenzoylmethane | 2,50% | 2,50% | 2,50% | 2,50% |
| Bis-Ethylhexyloxyphenol Methoxyphenyl Triazine¹⁵ | 5,50% | 5,50% | 5,50% | 5,50% |
| Tocopheryl Acetate | 0,50% | 0,50% | 0,50% | 0,50% |
| Glycerin | 2,00% | 2,00% | 2,00% | 2,00% |
| Wasser | ad 100% | ad 100% | ad 100% | ad 100% |
| Acrylates/C10-30 Alkyl Acrylate Crosspolymer¹⁶ | 0,10% | 0,10% | 0,10% | 0,10% |

| | | | | |
|---|---|---|---|---|
| ¹¹ SKINMIMICS^{®} (Evonik Nutrition & Care GmbH) ¹² SPHINGOKINE^{®} NP (Evonik Nutrition & Care GmbH) ¹³ Verstatil^{®} PC (Dr. Straetmans GmbH) ¹⁴ TEGOSOFT^{®} XC (Evonik Nutrition & Care GmbH) ¹⁵ Tinosorb S (BASF) ¹⁶ TEGO^{®} Carbomer 341 ER (Evonik Nutrition & Care GmbH) | | | | |

| | | | | |
|---|---|---|---|---|
| Sodium Hydroxide (10%ig in Wasser) | 0,30% | 0,30% | 0,30% | 0,30% |
| Dipropylene Glycol; Methylparaben; Ethylparaben; Aqua; Methylisothiazolinone¹⁷ | 0,80% | 0,80% | 0,80% | 0,80% |

### Lotion basierend auf natürlichen Inhaltsstoffen

| Rezeptur | 7-1 | 7-2 | 7-3 | 7-4 |
|---|---|---|---|---|
| Erfindungsgemäßes Rhamnolipidderivat nach Bsp. 4a | 2,50% | 1,50% | - | - |
| Erfindungsgemäßes Rhamnolipidderivat nach Bsp. 4b | - | - | 2,50% | 1,50% |
| Polyglyceryl-3 Dicitrate/Stearate¹⁸ | - | 1,00% | - | 1,00% |
| Isopropyl Palmitate | 5,00% | 5,00% | 5,00% | 5,00% |
| Prunus Amygdalus Dulcis (Sweet Almond) Oil | 5,00% | 5,00% | 5,00% | 5,00% |
| Wasser | ad 100% | ad 100% | ad 100% | ad 100% |
| Glycerin | 3,00% | 3,00% | 3,00% | 3,00% |
| Xanthan Gum⁷ | 0,50% | 0,50% | 0,50% | 0,50% |
| Sodium Hydroxide (10%ig in Wasser) | 0,20% | 0,20% | 0,20% | 0,20% |
| Benzyl Alcohol; Glycerin; Benzoic Acid; Sorbic Acid¹⁹ | 0,80% | 0,80% | 0,80% | 0,80% |

| | | | | |
|---|---|---|---|---|
| ¹⁷ Microcare MEM (Thor) ¹⁸ TEGO^{®} Care PSC 3 (Evonik Nutrition & Care GmbH) ¹⁹ Rokonsal BSB-N (Ashland) | | | | |

### Mittels Kaltherstellung erzeugte leichte O/W Lotion

| Rezeptur | 8-1 | 8-2 |
|---|---|---|
| Erfindungsgemäßes Rhamnolipidderivat nach Bsp. 3a | 2,50% | 1,50% |
| Sorbitan Laurate; Polyglyceryl-4 Laurate; Dilauryl Citrate⁵ | - | 1,00% |
| Ethylhexyl Palmitate | 1,10% | 1,10% |
| Isohexadecane | 5,50% | 5,50% |
| Cyclopentasiloxane | 5,00% | 5,00% |
| Cellulose²⁰ | 1,00% | 1,00% |
| Carbomer⁶ | 0,15% | 0,15% |
| Carbomer¹ | 0,15% | 0,15% |
| Xanthan Gum | 0,10% | 0,10% |
| Glycerin | 3,00% | 3,00% |
| Wasser | ad 100% | ad 100% |
| Sodium Hydroxide (10%ig in Wasser) | 0,90% | 0,90% |
| Phenoxyethanol; Ethylhexylglycerin² | 0,70% | 0,70% |

### O/W Baby Lotion

| Rezeptur | 9-1 | 9-2 |
|---|---|---|
| Erfindungsgemäßes Rhamnolipidderivat nach Bsp. 3a | 3,00% | 2,00% |
| Polyglyceryl-3 Methylglucose Distearate²¹ | - | 0,50 % |
| Stearyl Alcohol | 1,00% | 1,00% |
| Isopropyl Palmitate | 7,30 % | 7,30 % |
| Triisostearin | 4,00% | 4,00% |
| Squalane | 1,00% | 1,00% |
| Dimethicone²² | 0,50% | 0,50% |
| Wasser | ad 100% | ad 100% |
| Glycerin | 3,00% | 3,00% |
| Carbomer¹ | 0,20% | 0,20% |
| Sodium Hydroxide (10%ig in Wasser) | 0,60% | 0,60% |
| Sodium Lactate; Sodium PCA; Glycine; Fructose; Urea; Niacinamide; Inositol; Sodium Benzoate; Lactic Acid²³ | 3,00% | 3,00% |
| Phenoxyethanol; Caprylyl Glycol¹¹ | 1,00% | 1,00% |

| | | |
|---|---|---|
| ²⁰ TEGO^{®} Feel C 10 (Evonik Nutrition & Care GmbH) ²¹ TEGO^{®} Care 450 (Evonik Nutrition & Care GmbH) ²² ABIL^{®} 350 (Evonik Nutrition & Care GmbH) | | |

### Imprägnierungslotion für Feuchttücher zur Babypflege

| Rezeptur | 10-1 | 10-2 | 10-3 | 10-4 |
|---|---|---|---|---|
| Erfindungsgemäßes | 1,50% | 1,00% | - | - |
| Rhamnolipidderivat nach Bsp. 4a | | | | |
| Erfindungsgemäßes | - | - | 2,50% | 1,50% |
| Rhamnolipidderivat nach Bsp. 3b | | | | |
| Cetearyl Glucoside³ | - | 0,50 % | - | 0,50 % |
| Polyglyceryl-3 Caprate²⁴ | 0,50% | 0,50% | 0,50% | 0,50% |
| C12-15 Alkyl Benzoate | 5,00% | 5,00% | 5,00% | 5,00% |
| Mineral Oil | 5,00% | 5,00% | 5,00% | 5,00% |
| Gellan Gum²⁵ | 0,03% | 0,03% | 0,03% | 0,03% |
| Wasser | ad 100% | ad 100% | ad 100% | ad 100% |
| Glycerin | 2,00% | 2,00% | 2,00% | 2,00% |
| Acrylates/C10-30 Alkyl Acrylate Crosspolymer¹⁴ | 0,05% | 0,05% | 0,05% | 0,05% |
| Sodium Hydroxide (10%ig in Wasser) | 0,15% | 0,15% | 0,15% | 0,15% |
| Phenoxyethanol; Ethylhexylglycerin² | 0,70% | 0,70% | 0,70% | 0,70% |

### O/W Foundation für ein natürliches Hautgefühl

| Rezeptur | 11-1 | 11-2 | 11-3 | 11-4 |
|---|---|---|---|---|
| Erfindungsgemäßes | 5,00% | 3,00% | 5,00% | 3,00% |
| Rhamnolipidderivat nach Bsp. 3a | | | | |
| Polyglyceryl-6 Stearate (and) Polyglyceryl-6 Behenate⁴ | - | 1,50% | - | 1,50% |
| Cetearyl Alcohol | 1,50% | 1,50% | 1,50% | 1,50% |
| Glyceryl Stearate | 1,00% | 1,00% | 1,00% | 1,00% |

| | | | | |
|---|---|---|---|---|
| ²³ LACTIL^{®} (Evonik Nutrition & Care GmbH) ²⁴ TEGOSOFT^{®} PC 31 (Evonik Nutrition & Care GmbH) ²⁵ Kelcogel CG-HA (CP Kelco) | | | | |

| | | | | |
|---|---|---|---|---|
| Myristyl Myristate²⁶ | 1,00% | 1,00% | 1,00% | 1,00% |
| C12-15 Alkyl Benzoate | 3,00% | 3,00% | 3,00% | 3,00% |
| Diethylhexyl Carbonate²⁷ | 2,00% | 2,00% | 2,00% | 2,00% |
| Cyclopentasiloxane (and) Dimethicone Crosspolymer²⁸ | 2,00% | 2,00% | 2,00% | 2,00% |
| Ethylhexyl Methoxycinnamate | 8,00% | 8,00% | 8,00% | 8,00% |
| Ethylhexyl Salicylate | 5,00% | 5,00% | 5,00% | 5,00% |
| Diethylamino Hydroxybenzoayl Hexyl Benzoate²⁹ | 3,00% | 3,00% | 3,00% | 3,00% |
| Bis-Ethylhexyloxyphenol Methoxyphenyl Triazine¹³ | 3,00% | 3,00% | 3,00% | 3,00% |
| Wasser | ad 100% | ad 100% | ad 100% | ad 100% |
| Glycerin | 1,00% | 1,00% | 1,00% | 1,00% |
| Xanthan Gum | 0,15% | 0,15% | 0,15% | 0,15% |
| Titanium Dioxide; CI 77891 | 8,00% | 8,00% | 8,00% | 8,00% |
| CI 77492; Aqua; Glycerin; Xanthan Gum; Sodium Citrate³⁰ | 1,70% | 1,70% | 1,70% | 1,70% |
| CI 77491; Aqua; Glycerin; Xanthan Gum; Sodium Citrate³¹ | 0,40% | 0,40% | 0,40% | 0,40% |
| CI 77499; Aqua; Glycerin; Xanthan Gum; Sodium Citrate³² | 0,10% | 0,10% | 0,10% | 0,10% |
| Glycerin | 2,00% | 2,00% | 2,00% | 2,00% |
| Propylene Glycol | 3,00% | 3,00% | 3,00% | 3,00% |
| Nylon-12 | 2,00% | 2,00% | - | - |
| Cellulose¹⁸ | - | - | 2,00% | 2,00% |
| Phenoxyethanol; Ethylhexylglycerin² | 0,70% | 0,70% | 0,70% | 0,70% |

### O/W Lotion zur Make-up-Entfernung mit seidigem Hautgefühl

| Rezeptur | 12-1 | 12-2 |
|---|---|---|
| Erfindungsgemäßes | 3,00% | 2,00% |
| Rhamnolipidderivat nach Bsp. 3a | | |

| | | |
|---|---|---|
| ²⁶ TEGOSOFT^{®} MM (Evonik Nutrition & Care GmbH) ²⁷ TEGOSOFT^{®} DEC (Evonik Nutrition & Care GmbH) ²⁸ 9045 Silicone Elastomer Blend (Dow Corning) ²⁹ Uvinul A plus Granular (BASF) ³⁰ Covarine Yellow WN 1792 GZ (Sensient Cosmetic Technologies) ³¹ Covarine Red WN 3798 GZ (Sensient Cosmetic Technologies) ³² Covarine Black WN 9798 GZ (Sensient Cosmetic Technologies) | | |

| | | |
|---|---|---|
| Bis-PEG/PPG-20/5 PEG/PPG-20/5 | - | 1,00% |
| Dimethicone: Methoxy PEG/ PPG-25/4 Dimethicone; Caprylic/Capric Triglyceride³³ | | |
| Decyl Cocoate³⁴ | 7,00% | 7,00% |
| Paraffinum Perliquidum | 7,00% | 7,00% |
| Acrylates/C10-30 Alkyl Acrylate Crosspolymer¹⁴ | 0,15% | 0,15% |
| Glycerin | 3,00% | 3,00% |
| Wasser | ad 100% | ad 100% |
| Sodium Hydroxide (10%ig in Wasser) | 0,45% | 0,45% |
| Alcohol | 3,00% | 3,00% |
| Phenoxyethanol; Caprylyl Glycol¹¹ | 1,00% | 1,00% |

### Natürliche ölfreisetzende Creme

| Rezeptur | 13-1 | 13-2 | 14-1 | 14-2 |
|---|---|---|---|---|
| Erfindungsgemäßes | 4,00% | 2,00% | 4,00% | 2,00% |
| Rhamnolipidderivat nach Bsp. 4a | | | | |
| Polyglyceryl-3 Distearate; Glyceryl Stearate Citrate³⁵ | - | 1,00% | - | - |
| Polyglyceryl-6 Stearate; Polyglyceryl-6 Behenate | - | - | - | 1,00% |
| Decyl Cocoate³² | 72,00% | 72,00% | - | - |
| Helianthus Annuus (Sunflower) Seed Oil | | | 70,0% | 70,0% |
| Glycerin | 2,00% | 2,00% | 2,00% | 2,00% |
| Wasser | ad 100% | ad 100% | ad 100% | ad 100% |
| Benzyl Alcohol; Glycerin; Benzoic Acid; Sorbic Acid¹⁷ | 1,00% | 1,00% | 1,00% | 1,00% |
| Citric Acid (10% aq.) | q.s. | q.s. | q.s. | q.s. |

| | | | | |
|---|---|---|---|---|
| ³³ ABIL^{®} Care XL 80 (Evonik Nutrition & Care GmbH) ³⁴ TEGOSOFT^{®} DC (Evonik Nutrition & Care GmbH) ³⁵ TEGO^{®} Care NC (Evonik Nutrition & Care GmbH) | | | | |

### Niedrigviskose W/O Lotion

| Rezeptur | 15-1 | 15-2 | 16-1 | 16-2 |
|---|---|---|---|---|
| Polyglyceryl-4 | 2,50% | 2,50% | - | - |
| Diisostearate/Polyhyd roxystearate/Sebacate³⁶ | | | | |
| PEG/PPG-10/1 Dimethicone³⁷ | - | - | 1,50% | 1,50% |
| Erfindungsgemäßes Rhamnolipidderivat nach Bsp. 4a | 0,50% | 0,20% | 0,50% | 0,20% |
| Di-Rhamnolipid | - | 0,20% | - | 0,20% |
| Hydrogenated Castor Oil | 0,20% | 0,20% | 0,20% | 0,20% |
| Isopropyl Palmitate | 10,00% | 10,00% | - | - |
| Prunus Amygdalus Dulcis (Sweet Almond) Oil | 8,00% | 8,00% | - | - |
| Diethylhexyl Carbonate²⁵ | 7,00% | 7,00% | 10,00% | 10,00% |
| Ethylhexyl Palmitate | - | - | 8,00% | 8,00% |
| Dimethicone (5 mPas) | - | - | 5,00% | 5,00% |
| Glycerin | 3,00% | 3,00% | 2,00% | 2,00% |
| Wasser | ad 100% | ad 100% | ad 100% | ad 100% |
| Magnesium Sulfate Heptahydrate | 1,50% | 1,50% | - | - |
| Sodium Chloride | - | - | 0,80% | 0,80% |
| Benzyl Alcohol; Glycerin; Benzoic Acid; Sorbic Acid¹⁷ | 1,00% | 1,00% | 1,00% | 1,00% |
| Citric Acid (10% aq.) | q.s. | q.s. | q.s. | q.s. |

| | | | | |
|---|---|---|---|---|
| ³⁶ ISOLAN^{®} GPS (Evonik Nutrition & Care GmbH) ³⁷ ABIL^{®} EM 90 (Evonik Nutrition & Care GmbH) | | | | |

## Patentansprüche

1. Dispersion enthaltend mindestens A) ein Rhamnolipidderivat ausgewählt aus der Gruppe bestehen aus Rhamnolipidester der allgemeinen Formel (I) und Rhamnolipidamide der allgemeinen Formel (II): 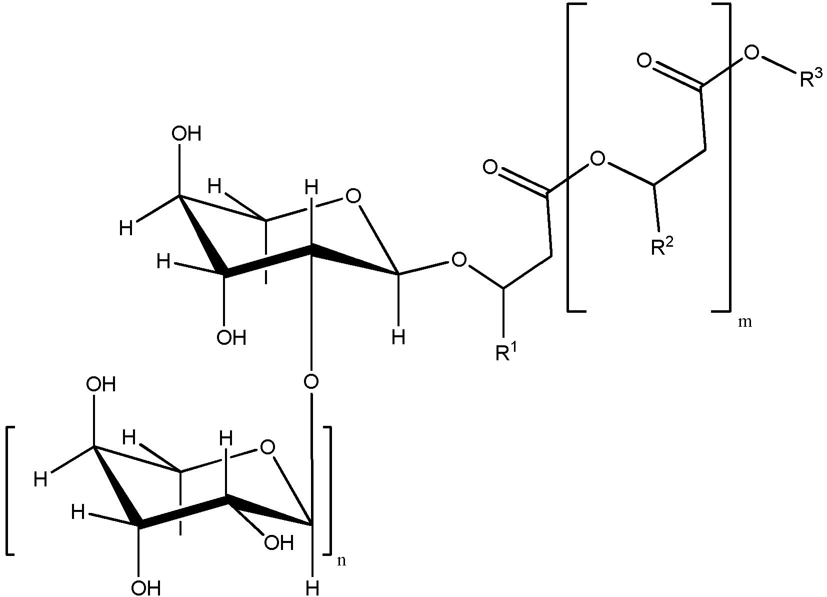 allgemeine Formel (I),
wobei
m = 2, 1 oder 0, insbesondere 1 oder 0,
n = 1 oder 0, insbesondere 1,
R¹ = organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12, und
R³ = aliphatischer Rest mit 7 bis 32 Kohlenstoffatomen, bevorzugt 8 bis 24 Kohlenstoffatomen, besonders bevorzugt 10 bis 22 Kohlenstoffatomen, welcher ausgewählt ist aus der Gruppe der Reste R³ direkt abgeleitet von R³OH = natürlicher Fettalkohol, 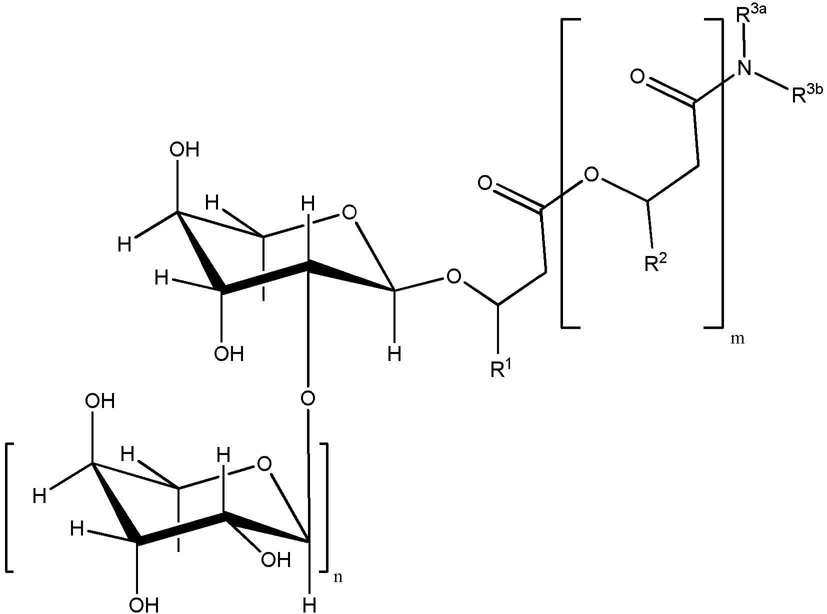
allgemeine Formel (II),
wobei
m = 2, 1 oder 0, insbesondere 1 oder 0,
n = 1 oder 0, insbesondere 1,
R¹ = organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
R^{3a} = organischer Rest mit 1 bis 32, bevorzugt 8 bis 24, besonders bevorzugt 10 bis 22, Kohlenstoffatomen,
und R^{3b} = organischer Rest mit 1 bis 32, bevorzugt 8 bis 24, besonders bevorzugt 10 bis 22, Kohlenstoffatomen oder H, bevorzugt H,
mit der Maßgabe, dass die Summe der in R^{3a} und R^{3b} enthaltenen Kohlenstoffatome 7 bis 44, bevorzugt 8 bis 24, besonders bevorzugt 10 bis 22, beträgt.

2. Dispersion gemäß Anspruch 1, **dadurch gekennzeichnet dass** sie eine Emulsion darstellt.

3. Dispersion gemäß Anspruch 1 oder 2, **dadurch gekennzeichnet dass** sie
A) das Rhamnolipidderivat,
B) mindestens ein kosmetisches Öl und
C) Wasser
enthält.

4. ) Dispersion gemäß Anspruch 3, **dadurch gekennzeichnet, dass**
A) in einer Menge von 0,1 Gew.-% bis 10,0 Gew.-%, bevorzugt in einer Menge von 0,5 Gew.-% bis 7,0 Gew.-%, besonders bevorzugt in einer Menge von 1,0 Gew.-% bis 5,0 Gew.-%,
B) in einer Menge von 5,0 Gew.-% bis 79,9 Gew.-%, bevorzugt in einer Menge von 10,0 Gew.-% bis 50,0 Gew.-%, besonders bevorzugt in einer Menge von 12,0 Gew.-% bis 35,0 Gew.-%,
C) in einer Menge von 20,0 Gew.-% bis 94,9 Gew.-%, bevorzugt in einer Menge von 50,0 Gew.-% bis 90,0 Gew.-%, besonders bevorzugt in einer Menge von 65,0 Gew.-% bis 88,0 Gew.-%,
enthalten ist, wobei sich die Gew.-% auf die Gesamtdispersion beziehen.

5. ) Dispersion gemäß mindestens einem der vorherigen Ansprüche, **dadurch gekennzeichnet, dass** bei dem enthaltenen Rhamnolipidester R³ ausgewählt ist aus verzweigten oder linearen Alkylresten, bevorzugt mit 8 bis24, insbesondere 10 bis 22, Kohlenstoffatomen.

6. ) Dispersion gemäß mindestens einem der vorherigen Ansprüche, **dadurch gekennzeichnet, dass** bei dem enthaltenen Rhamnolipidester R³ ausgewählt ist aus der Gruppe umfassend, bevorzugt bestehend aus, Lauryl-, Myristyl-, Palmityl- und Stearyl-, Arachidyl- und Behenylresten.

7. ) Dispersion gemäß mindestens einem der vorherigen Ansprüche, **dadurch gekennzeichnet, dass** bei dem enthaltenen Rhamnolipidamid R^{3a} ausgewählt ist aus der Gruppe der Alkylreste, welche gegebenenfalls Amingruppe aufweisen, und R^{3b} = Alkylrest mit 1 bis 8 Kohlenstoffatomen oder H, bevorzugt H.

8. ) Dispersion gemäß mindestens einem der vorherigen Ansprüche, **dadurch gekennzeichnet, dass** bei dem enthaltenen Rhamnolipidamid R^{3a} ausgewählt ist aus der Gruppe umfassend, 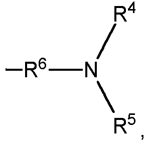 wobei
R⁴ und R⁵ = unabhängig voneinander gleicher oder verschiedener Alkylrest mit 1 bis 6, bevorzugt 1 bis 3, besonders bevorzugt 1, Kohlenstoffatomen,
R⁶ = eine Alkylengruppe mit 1 bis 6, bevorzugt, 2 bis 3 Kohlenstoffatomen,
und 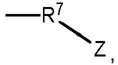 wobei
R⁷ = eine Alkylengruppe mit 1 bis 22, bevorzugt, 2 bis 18, insbesondere 3 bis 8 Kohlenstoffatomen,
Z = H, OH, OR⁸ mit
R⁸ = Alkylrest mit 1 bis 6, bevorzugt 1 bis 3, besonders bevorzugt 1, Kohlenstoffatomen,
und bevorzugt
R^{3b} = H.

9. ) Dispersion gemäß mindestens einem der vorherigen Ansprüche, **dadurch gekennzeichnet, dass** bei dem enthaltenen Rhamnolipidamid sich der Rest -NR^{3a}R^{3b} ableitet von einem Amin NHR^{3a}R^{3b} ausgewählt aus Aminosäuren und Peptiden.

10. ) Verwendung der Rhamnolipidderivate genannt in einem der vorherigen Ansprüche als Emulgator oder Dispergierhilfsmittel.

## Claims

1. Dispersion containing at least A) one rhamnolipid derivative selected from the group consisting of rhamnolipid ester of the general formula (I) and rhamnolipid amide of the general formula (II): 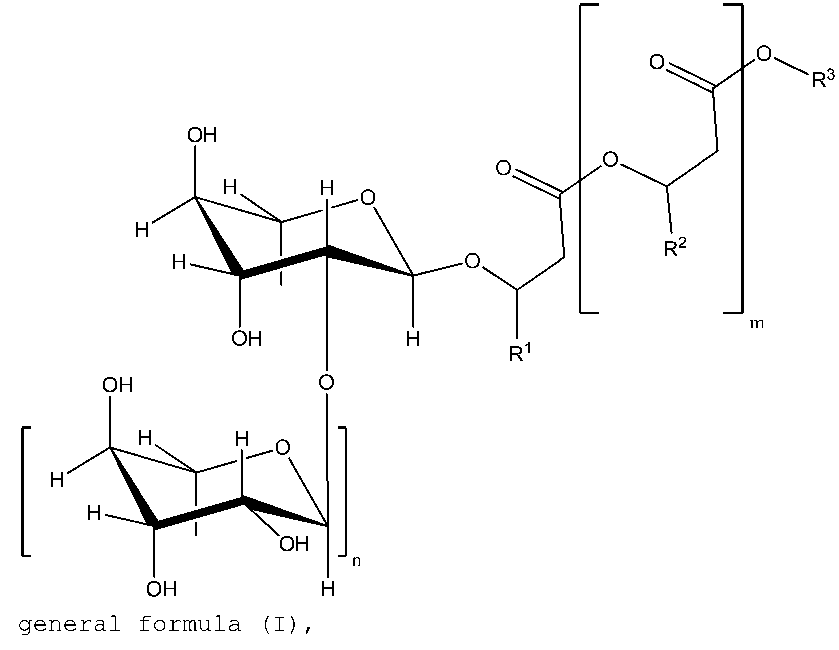 where
m = 2, 1 or 0, in particular 1 or 0,
n = 1 or 0, in particular 1,
R¹ = organic radical having 2 to 24, preferably 5 to 13, carbon atoms, in particular optionally branched, optionally substituted, in particular hydroxy-substituted, optionally unsaturated, in particular optionally mono-, bi- or tri-unsaturated, alkyl radical, preferably one selected from the group consisting of pentenyl, heptenyl, nonenyl, undecenyl and tridecenyl and (CH₂)ₒ-CH₃ where o = 1 to 23, preferably 4 to 12,
R² = independently of one another, identical or different, organic radical having 2 to 24, preferably 5 to 13, carbon atoms, in particular optionally branched, optionally substituted, in particular hydroxy-substituted, optionally unsaturated, in particular optionally mono-, bi- or tri-unsaturated, alkyl radical, preferably one selected from the group consisting of pentenyl, heptenyl, nonenyl, undecenyl and tridecenyl and (CH₂)ₒ-CH₃ where o = 1 to 23, preferably 4 to 12, and
R³ = aliphatic radical having 7 to 32 carbon atoms, preferably 8 to 24 carbon atoms, more preferably 10 to 22 carbon atoms, which is selected from the group of R³ radicals derived directly from R³OH = natural fatty alcohol, 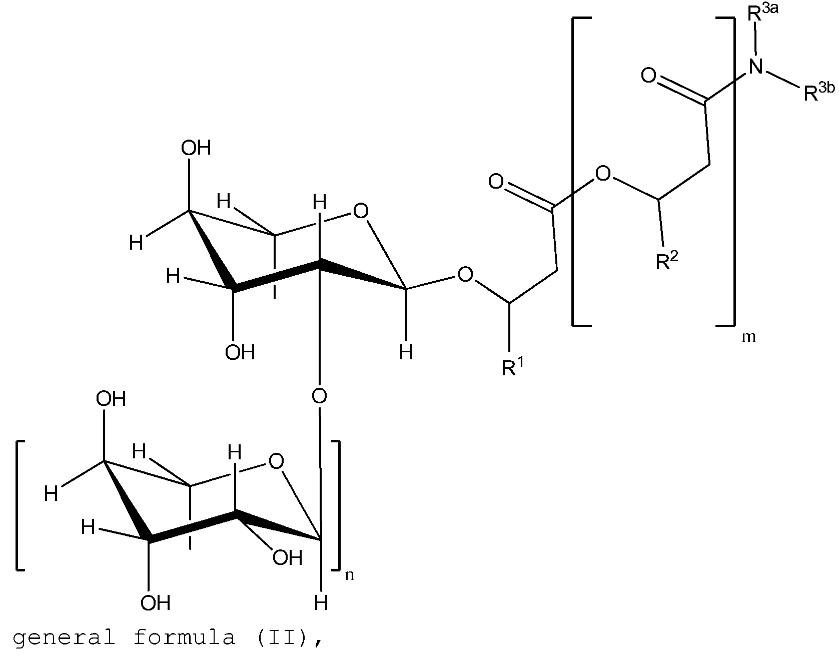 where
m = 2, 1 or 0, in particular 1 or 0,
n = 1 or 0, in particular 1,
R¹ = organic radical having 2 to 24, preferably 5 to 13, carbon atoms, in particular optionally branched, optionally substituted, in particular hydroxy-substituted, optionally unsaturated, in particular optionally mono-, bi- or tri-unsaturated, alkyl radical, preferably one selected from the group consisting of pentenyl, heptenyl, nonenyl, undecenyl and tridecenyl and (CH₂)ₒ-CH₃ where o = 1 to 23, preferably 4 to 12,
R² = independently of one another, identical or different, organic radical having 2 to 24, preferably 5 to 13, carbon atoms, in particular optionally branched, optionally substituted, in particular hydroxy-substituted, optionally unsaturated, in particular optionally mono-, bi- or tri-unsaturated, alkyl radical, preferably one selected from the group consisting of pentenyl, heptenyl, nonenyl, undecenyl and tridecenyl and (CH₂)ₒ-CH₃ where o = 1 to 23, preferably 4 to 12,
R^{3a} = organic radical having 1 to 32, preferably 8 to 24, particularly preferably 10 to 22, carbon atoms,
and R^{3b} = organic radical having 1 to 32, preferably 8 to 24, particularly preferably 10 to 22, carbon atoms or H, preferably H,
with the proviso that the sum of the carbon atoms contained in R^{3a} and R^{3b} is 7 to 44, preferably 8 to 24, particularly preferably 10 to 22.

2. Dispersion according to Claim 1, **characterized in that** it is an emulsion.

3. Dispersion according to Claim 1 or 2, **characterized in that** it contains
A) the rhamnolipid derivative,
B) at least one cosmetic oil, and
C) water.

4. Dispersion according to Claim 3, **characterized in that** it contains
A) in an amount of 0.1 wt% to 10.0 wt%, preferably in an amount of 0.5 wt% to 7.0 wt%, more preferably in an amount of 1.0 wt% to 5.0 wt%,
B) in an amount of 5.0 wt% to 79.9 wt%, preferably in an amount of 10.0 wt% to 50.0 wt%, more preferably in an amount of 12.0 wt% to 35.0 wt%,
C) in an amount of 20.0 wt% to 94.9 wt%, preferably in an amount of 50.0 wt% to 90.0 wt%, more preferably in an amount of 65.0 wt% to 88.0 wt%,
where the percentages by weight refer to the total dispersion.

5. Dispersion according to at least one of the preceding claims, **characterized in that**, in the rhamnolipid ester present, R³ is selected from branched or linear alkyl radicals, preferably with 8 to 24, in particular 10 to 22, carbon atoms.

6. Dispersion according to at least one of the preceding claims, **characterized in that**, in the rhamnolipid ester present, R³ is selected from the group comprising, preferably consisting of, lauryl, myristyl, palmityl, stearyl, arachidyl and behenyl radicals.

7. Dispersion according to at least one of the preceding claims, **characterized in that**, in the rhamnolipid amide present, R^{3a} is selected from the group of the alkyl radicals which optionally have amine groups, and R^{3b} = alkyl radical with 1 to 8 carbon atoms or H, preferably H.

8. Dispersion according to at least one of the preceding claims, **characterized in that**, in the rhamnolipid amide present, R^{3a} is selected from the group comprising 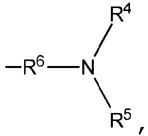 where
R⁴ and R⁵ = independently of one another, identical or different alkyl radical with 1 to 6, preferably 1 to 3, particularly preferably 1, carbon atom(s),
R⁶ = an alkylene group with 1 to 6, preferably 2 to 3, carbon atoms,
and 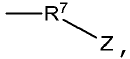 where
R⁷ = an alkylene group with 1 to 22, preferably 2 to 18, in particular 3 to 8, carbon atoms,
Z = H, OH, OR⁸, where
R⁸ = alkyl radical with 1 to 6, preferably 1 to 3, particularly preferably 1, carbon atom(s),
and preferably
R^{3b} = H.

9. Dispersion according to at least one of the preceding claims, **characterized in that**, in the rhamnolipid amide present, the radical -NR^{3a}R^{3b} is derived from an amine NHR^{3a}R^{3b}, selected from amino acids and peptides.

10. Use of the rhamnolipid derivatives mentioned in one of the preceding claims, as emulsifier or dispersing aid.

## Revendications

1. Dispersion contenant au moins A) un dérivé de rhamnolipide choisi dans le groupe constitué par les esters de rhamnolipide de formule générale (I) et les amides de rhamnolipide de formule générale (II) : 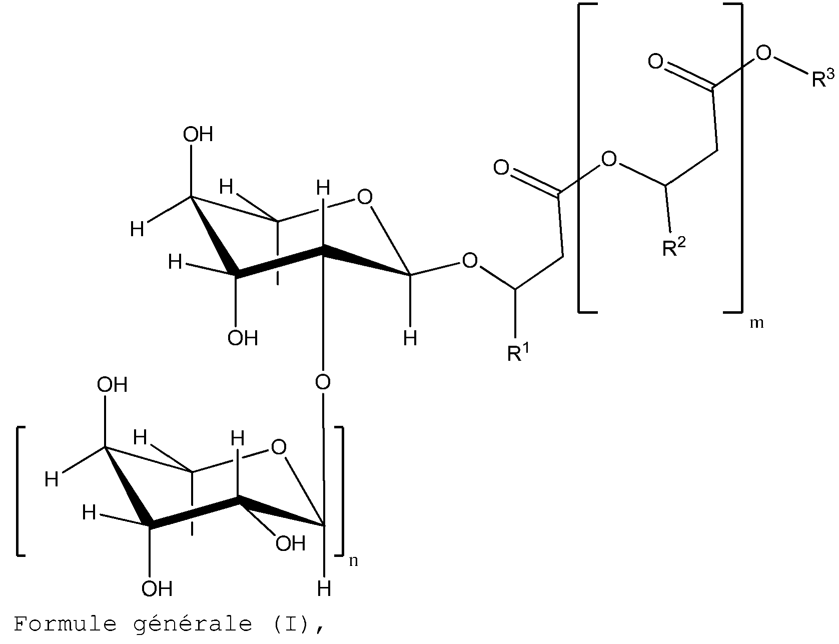 dans laquelle
m = 2, 1 ou 0, en particulier 1 ou 0,
n = 1 ou 0, en particulier 1,
R¹ = un radical organique comprenant 2 à 24, de préférence 5 à 13 atomes de carbone, en particulier un radical alkyle le cas échéant ramifié, le cas échéant substitué, en particulier substitué par hydroxy, le cas échéant insaturé, en particulier le cas échéant mono-insaturé, bi-insaturé ou tri-insaturé, de préférence un tel radical choisi dans le groupe pentényle, heptényle, nonényle, undécényle et tridécényle et (CH₂)ₒ-CH₃ où o = 1 à 23, de préférence 4 à 12,
R² = indépendamment, un radical organique identique ou différent, comprenant 2 à 24, de préférence 5 à 13 atomes de carbone, en particulier un radical alkyle le cas échéant ramifié, le cas échéant substitué, en particulier substitué par hydroxy, le cas échéant insaturé, en particulier le cas échéant mono-insaturé, bi-insaturé ou tri-insaturé, de préférence un tel radical choisi dans le groupe constitué pentényle, heptényle, nonényle, undécényle et tridécényle et (CH₂)ₒ-CH₃ où o = 1 à 23, de préférence 4 à 12, et
R³ = radical aliphatique comprenant 7 à 32 atomes de carbone, de préférence 8 à 24 atomes de carbone, de manière particulièrement préférée 10 à 22 atomes de carbone, qui est choisi dans le groupe des radicaux R³ directement dérivés de R³OH = alcool gras naturel, 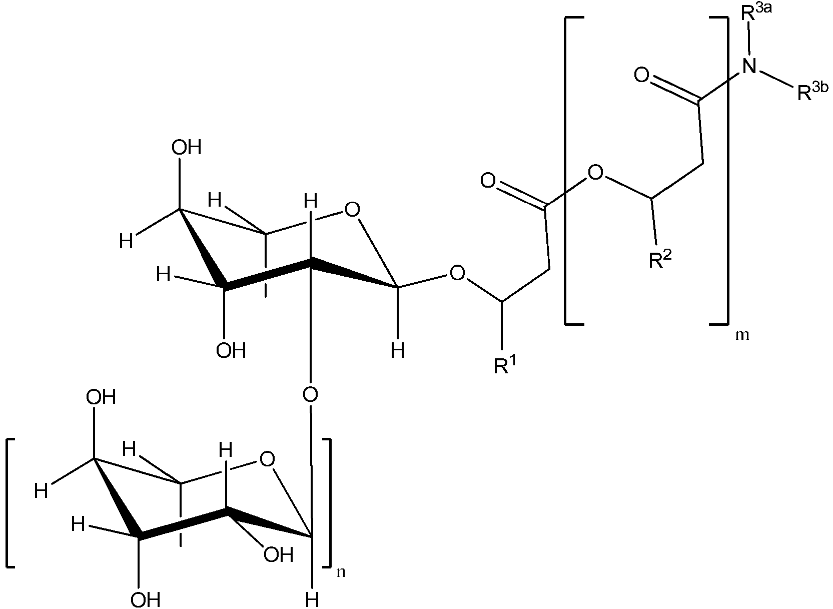 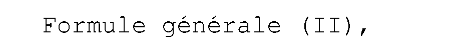 dans laquelle
m = 2, 1 ou 0, en particulier 1 ou 0,
n = 1 ou 0, en particulier 1,
R¹ = un radical organique comprenant 2 à 24, de préférence 5 à 13 atomes de carbone, en particulier un radical alkyle le cas échéant ramifié, le cas échéant substitué, en particulier substitué par hydroxy, le cas échéant insaturé, en particulier le cas échéant mono-insaturé, bi-insaturé ou tri-insaturé, de préférence un tel radical choisi dans le groupe pentényle, heptényle, nonényle, undécényle et tridécényle et (CH₂)ₒ-CH₃ où o = 1 à 23, de préférence 4 à 12,
R² = indépendamment, un radical organique identique ou différent, comprenant 2 à 24, de préférence 5 à 13 atomes de carbone, en particulier un radical alkyle le cas échéant ramifié, le cas échéant substitué, en particulier substitué par hydroxy, le cas échéant insaturé, en particulier le cas échéant mono-insaturé, bi-insaturé ou tri-insaturé, de préférence un tel radical choisi dans le groupe constitué pentényle, heptényle, nonényle, undécényle et tridécényle et (CH₂)ₒ-CH₃ où o = 1 à 23, de préférence 4 à 12,
R^{3a} = un radical organique comprenant 1 à 32, de préférence 8 à 24, de manière particulièrement préférée 10 à 22, atomes de carbone,
et R^{3b} = radical organique comprenant 1 à 32, de préférence 8 à 24, de manière particulièrement préférée 10 à 22, atomes de carbone, ou H, de préférence H,
sous réserve que la somme des atomes de carbone contenus dans R^{3a} et R^{3b} représente 7 à 44, de préférence 8 à 24, de manière particulièrement préférée 10 à 22.

2. Dispersion selon la revendication 1, **caractérisée en ce qu'**il s'agit d'une émulsion.

3. Dispersion selon la revendication 1 ou 2, **caractérisée en ce qu'**elle contient
A) le dérivé de rhamnolipide
B) au moins une huile cosmétique et
C) de l'eau

4. Dispersion selon la revendication 3, **caractérisée en ce que**
A) est contenu en une quantité de 0,1% en poids à 10,0% en poids, de préférence en une quantité de 0,5% en poids à 7,0% en poids, de manière particulièrement préférée en une quantité de 1,0% en poids à 5,0% en poids,
B) est contenu en une quantité de 5,0% en poids à 79,9% en poids, de préférence en une quantité de 10,0% en poids à 50,0% en poids, de manière particulièrement préférée en une quantité de 12,0% en poids à 35,0% en poids,
C) est contenu en une quantité de 20,0% en poids à 94,9% en poids, de préférence en une quantité de 50,0% en poids à 90,0% en poids, de manière particulièrement préférée en une quantité de 65,0% en poids à 88,0% en poids,
les % en poids se référant à la dispersion totale.

5. Dispersion selon au moins l'une des revendications précédentes, **caractérisée en ce que** dans l'ester de rhamnolipide contenu, R³ est choisi parmi les radicaux alkyle ramifiés ou linéaires, comprenant de préférence 8 à 24, en particulier 10 à 22 atomes de carbone.

6. Dispersion selon au moins l'une des revendications précédentes, **caractérisée en ce que** dans l'ester de rhamnolipide contenu, R³ est choisi dans le groupe comprenant, de préférence constitué par, les radicaux lauryle, myristyle, palmityle et stéaryle, arachidyle et béhényle.

7. Dispersion selon au moins l'une des revendications précédentes, **caractérisée en ce que** dans l'amide de rhamnolipide contenu, R^{3a} est choisi dans le groupe des radicaux alkyle, qui présentent le cas échéant un groupe amine, et R^{3b} = radical alkyle comprenant 1 à 8 atomes de carbone ou H, de préférence H.

8. Dispersion selon au moins l'une des revendications précédentes, **caractérisée en ce que** dans l'amide de rhamnolipide contenu, R^{3a} est choisi dans le groupe comprenant 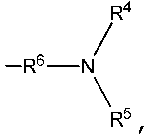 dans laquelle
R⁴ et R⁵ = indépendamment l'un de l'autre, des radicaux alkyle identiques ou différents comprenant 1 à 6, de préférence 1 à 3, de manière particulièrement préférée 1, atomes de carbone,
R⁶ = un groupe alcényle comprenant 1 à 6, de préférence 2 à 3 atomes de carbone,
et 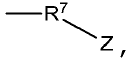 dans laquelle
R⁷ = représente un groupe alcényle comprenant 1 à 22, de préférence 2 à 18, en particulier de 3 à 8 atomes de carbone
Z = H, OH, OR⁸ où
R⁸ = un radical alkyle comprenant 1 à 6, de préférence 1 à 3, de manière particulièrement préférée 1, atomes de carbone,
et de préférence
R^{3b} = H.

9. Dispersion selon au moins l'une des revendications précédentes, **caractérisée en ce que** dans l'amide de rhamnolipide contenu, le radical -NR^{3a}R^{3b} est dérivé d'une amine NHR^{3a}R^{3b} choisie parmi les acides aminés et les peptides.

10. Utilisation des dérivés de rhamnolipide selon l'une des revendications précédentes en tant qu'émulsifiant ou adjuvant de dispersion.
